# EUROPÄISCHE PATENTANMELDUNG

(11) **EP 4 732 802 A1**
(43) Veröffentlichungstag der Anmeldung: **29.04.2026**
(21) Anmeldenummer: 24208705.4
(22) Anmeldetag: 24.10.2024
(51) Int. Cl.: A61B 34/20, A61B 17/00

(54) **KONTEXTINFORMATION FÜR GESCHÄTZTE PATIENTEN-REGISTRIERUNG BASIEREND AUF ÖRTLICHER REFERENZ**

(71) Anmelder: Carl Zeiss Meditec AG, 07745 Jena (DE); Universität Bern, 3012 Bern (CH)
(72) Erfinder: RAABE, Andreas, 3010 Bern (CH); BACHER, Neal, 73447 Oberkochen (DE); SAUR, Stefan, 73447 Oberkochen (DE)
(74) Vertreter: Kraus & Lederer PartGmbB

(57) **Zusammenfassung**

Es werden Techniken zur Bestimmung von Kontextinformation für eine geschätzte Patienten-Registrierung (85) beschrieben. Die Patienten-Registrierung kann z.B. von einem chirurgischen Navigationssystem geschätzt werden. Die Kontextinformation kann z.B. eine Unsicherheit für die Patienten-Registrierung und/oder eine alternative Schätzung für die Patienten-Registrierung angeben. Die Kontextinformation wird anhand von intraoperativen Bilddaten, die mittels eines chirurgischen Visualisierungssystems erfasst werden, bestimmt. Basierend auf der Kontextinformation können dann ein oder mehrere Komponenten, die mit einem chirurgischen Assistenzsystem assoziiert sind, angesteuert werden.

## Beschreibung

### TECHNISCHES GEBIET

Nachfolgend werden Techniken beschrieben, um Kontextinformation für eine indirekte Schätzung einer Patienten-Registrierung zwischen einem lokalen Patienten-Koordinatensystem und einem globalen Referenz-Koordinatensystem während eines operativen Eingriffs zu bestimmen. Die Kontextinformation kann zum Beispiel eine Bewertung der Schätzung der Patienten-Registrierung, etwa im Zusammenhang mit einer Unsicherheit, bereitstellen oder eine alternative Schätzung der Patienten-Registrierung.

### HINTERGRUND

Bei operativen Eingriffen kann es erstrebenswert sein, mittels eines chirurgischen Visualisierungssystems (insbesondere eines klassischen oder digitalen oder hybriden Operationsmikroskops) erfasste intraoperative Bilddaten mit präoperativen Bilddaten überlagert darzustellen. Beispielsweise kann derart eine Assistenzfunktionalität, zum Beispiel eine Führung des operativen Eingriffs hin zu einer Zielregion - z.B. ein Tumor oder Aneurysma im Rahmen der kranialen Chirurgie -, ermöglicht werden. Dazu ist es notwendig, die mittels des Operationsmikroskops erfassten intraoperativen Bilddaten mit den präoperativen Bilddaten derart zu überlagen, dass gleiche anatomische Strukturen deckungsgleich und mit richtiger Perspektive abgebildet werden.

Eine Möglichkeit besteht darin, anhand einer Analyse der intraoperativen Bilddaten und der präoperativen Bilddaten direkt eine Transformation zwischen den intraoperativen Bilddaten und den präoperativen Bilddaten zu bestimmen. Beispielsweise können in den intraoperativen Bilddaten und in den präoperativen Bilddaten bestimmte charakteristische Merkmale gesucht werden und aufeinander abgebildet werden. In einem solchen Fall ist keine Kenntnis der Lage des Patienten in einem globalen Referenz-Koordinatensystem, in dem auch eine Lage der Objekteben des Operationsmikroskops bekannt ist, notwendig. Allerdings weist eine solche Bild-zu-Bild-Transformation bestimmte Nachteile auf. Grundsätzlich ist eine solche rein bildbasierte Technik häufig wenig robust, weil die intraoperativen Bilddaten typischerweise nur zweidimensional (gegebenenfalls mit Tiefeninformation) vorliegen; aber keine Volumeninformationen beinhalten. Dies kann zu Mehrdeutigkeiten bei der Berechnung der Bild-zu-Bild-Transformation führen. Ferner kann es vorkommen, dass bestimmte für die Berechnung der Transformation verwendeten charakteristischen Merkmale temporär in den intraoperativen Bilddaten nicht sichtbar sind, z.B. weil sie verdeckt werden. Dann schlägt eine entsprechende Berechnung der Bild-zu-Bild-Transformation fehl. Ferner ist der Bildbereich - besonders bei mikrochirurgischen Eingriffen - begrenzt, so dass kleine Fehler in der Bild-zu-Bild Transformation schon erhebliche Ungenauigkeiten verursachen können.

Deshalb wird es typischerweise vorgezogen, ein lokales Patienten-Koordinatensystem in einem Beobachtungszeitraum während des operativen Eingriffs mit einem globalen Referenz-Koordinatensystem zu registrieren (es wird also eine Patienten-Registrierung bestimmt). In einem solchen Fall ist die Position und Orientierung (Position und Orientierung werden gemeinsam als Lage bezeichnet) des Patienten im globalen Referenz-Koordinatensystem während des Beobachtungszeitraums bekannt. Gleichzeitig kann auch die Lage des Operationsmikroskops im globalen Referenz-Koordinatensystem bestimmt und nachverfolgt werden. Dann kann auf die Perspektive des Operationsmikroskops auf den Patienten zurückgeschlossen werden. Insbesondere kann die Lage der Objektebene, die von den intraoperativen Bilddaten abgebildet wird, zurückgeschlossen werden. Derart kann eine genaue Überlagerung der intraoperativen Bilddaten mit den präoperativen Bilddaten erfolgen.

Eine solche Registrierung verschiedener Komponenten und insbesondere des Patienten in einem gemeinsamen globalen Referenz-Koordinatensystem wird typischerweise von einem Navigationssystem durchgeführt. Das Navigationssystem hat die Aufgabe, eine Schätzung der Patienten-Registrierung zu bestimmen.

Der entsprechende Ablauf zum Bestimmen der Schätzung der Patienten-Registrierung kann zweistufig erfolgen: zunächst findet eine (i) Initialisierungsphase statt; und (ii) anschließend eine Anpassungsphase. Beispielsweise kann in der (i) Initialisierungsphase die Hautoberfläche des Patienten in der Eingriffsregion direkt im globalen Referenz-Koordinatensystem vermessen und lokalisiert werden. Dazu kann z.B. die eine optische Abtastung mittels Laser erfolgen. Die Position des Laserspots kann im Referenz-Koordinatensystem mittels des Navigationssystems gemessen werden. Eine Abtastung könnte auch anders erfolgen, z.B. mit Berührung durch einen Pointer. Es könnten auch gezielt bestimmte anatomische Landmarken durch den Chirurgen markiert werden. Die Topographie der Anatomie des Patienten im Bereich der Eingriffsregion kann damit direkt im globalen Referenz-Koordinatensystem gemessen werden. In anderen Worten kann die Patienten-Registrierung initial direkt und absolut bestimmt werden. Während des chirurgischen Eingriffs ist es aber typischerweise unpraktisch oder unmöglich, die in der Initialisierungsphase zur Bestimmung der Patienten-Registrierung verwendete Messmodalität zu verwenden. Der Patient ist typischerweise durch Tücher abgedeckt und damit nicht oder nur eingeschränkt für die initiale Registrierung zu erreichen. Denn die entsprechende Vermessung der Topographie der Oberfläche des Patienten ist relativ aufwendig und benötigt eine Unterbrechung des chirurgischen Eingriffs. Deshalb wird eine solche Initialisierungsphase bevorzugt lediglich einmal zu Beginn eines chirurgischen Eingriffs durchgeführt, jedoch nicht wiederholt. Stattdessen wird - ausgehend von der Initialisierungsphase - die Anpassungsphase durchgeführt. Darin wird die Patienten-Registrierung von Zeit zu Zeit angepasst, um zum Beispiel eine Patientenbewegung zu kompensieren. In der Anpassungsphase erfolgt aber keine direkte Bestimmung der Patienten-Registrierung; vielmehr wird die Patienten-Registrierung geschätzt. Die Schätzung der Patienten-Registrierung tritt dann anstelle der direkt gemessenen initialen Patienten-Registrierung.

Im Stand der Technik sind unterschiedliche Verfahren zum Bestimmen einer Schätzung der Patienten-Registrierung in der Anpassungsphase bekannt.

Beispielsweise sind aus US 2019/0066314 A1 Techniken bekannt, bei denen eine sowohl in den intraoperativen Bilddaten wie auch in den präoperativen Bilddaten sichtbare Landmarke in den intraoperativ erfassten Bilddaten nachverfolgt wird und basierend auf der Änderung der Position der anatomischen Landmarke die initiale Registrierung des Patienten-Koordinatensystems und des Bilddaten-Koordinatensystems angepasst wird. Ein dort genanntes Beispiel für eine anatomische Landmarke ist eine Kraniotomie (Schädelöffnung). Eine solche Technik weist allerdings den Nachteil auf, dass manche anatomische Landmarke nicht während des gesamten chirurgischen Eingriffs sichtbar verbleiben. Beispielsweise könnte die Öffnung der Schädeldecke während des chirurgischen Eingriffs teilweise abgedeckt werden oder in den intraoperative erfassten Bilddaten eines Operationsmikroskops aufgrund der verwendeten Vergrößerung nicht oder nicht vollständig sichtbar sein.

Um entsprechende Nachteile der eingeschränkten Sichtbarkeit von anatomischen Landmarken zu vermeiden, werden in anderen im Stand der Technik bekannten Ansätzen künstliche Marker verwendet, die zum Beispiel mit einer Schraube an einem Knochen des Patienten befestigt sind und sich weg vom Knochen des Patienten hin zu einer exponierten Lage erstrecken. Diese künstlichen Marker sind grundsätzlich nicht in den präoperativen Bilddaten sichtbar, sondern werden erst im Vorfeld des operativen Eingriffs am Patienten befestigt oder notfallmäßig während des operativen Eingriffs. Derart wird eine besonders gute Sichtbarkeit, beispielsweise für die Stereokamera eines Navigationssystems, gewährleistet. Solche Techniken weisen allerdings den Nachteil auf, dass sie invasiv sind; das bedeutet, dass eine Bohrung angebracht werden muss, die den Knochen des Patienten beschädigt.

Als weitere im Stand der Technik bekannte Alternative wird in der Anpassungsphase eine Halterung verwendet, in der der Patient lösbar fixiert wird. Dabei ist also keine Knochenbohrung o. ä. notwendig. Beispielsweise kann der Patient in einer Halterung fixiert werden. Ein Beispiel für eine solche Halterung ist zum Beispiel eine Mayfield-Klemme, die am Kopf des Patienten angebracht wird. An der Halterung sind dann vom Navigationssystem lokalisierbare Marker angebracht. Die Lage der Marker wird im globalen Referenz-Koordinatensystem vom Navigationssystem gemessen. Basierend auf der Änderung der Lage der Marker kann die Änderung der Patienten-Registrierung geschätzt werden. Einfach gesprochen: Verändert sich die Lage der Marker im Referenz-Koordinatensystem, so kann angenommen werden, dass sich die Lage des Patienten-Koordinatensystems im globalen Referenz-Koordinatensystem entsprechend geändert hat.

Gegenüber einer solchen initial gemessenen und ggf. durch indirekte Lageverfolgung von an einer Halterung angebrachten Markern angepasste Schätzung der Patienten-Registrierung können während des chirurgischen Eingriffs zwei Arten von Veränderungen (Shifts) auftreten, die die Genauigkeit der Schätzung der Patienten-Registrierung beeinflussen:
Brainshift / Gewebedeformationen: Nicht-rigide Veränderungen zwischen einzelnen anatomischen Strukturen können durch chirurgische Manipulationen oder andere Faktoren wie z.B. Blutungen oder Schwellungen oder Austrocknung des Gehirns auftreten. Diese Deformationen führen zu einer räumlichen Änderung der Position von Gewebe und Strukturen im Patienten-Koordinatensystem. Ein weiterer Faktor für den Brainshift ist z.B. die Schädelöffnung selbst, da dann der craniale Druck nachlässt.

Schleichender Shift: Eine rigide (nur Translation und Rotation) Veränderung der Lage des Patienten gegenüber der Lage der Halterung kann durch äußere Einflüsse wie z.B. Gravitation, versehentliche Kollisionen oder Zugkräfte der Muskulatur des Patienten auftreten. Diese Veränderung führt zu einer Verschiebung des Patienten-Koordinatensystems gegenüber den nachverfolgten Markern, die an der Halterung befestigt sind. Insbesondere können sich entsprechende systematische Fehler über der Zeit akkumulieren und sind daher signifikant, jedoch für den Chirurgen aufgrund des schleichenden Charakters nur schwer zu erkennen.

In beiden Fällen ist es hilfreich, die Schätzung der Patienten-Registrierung anzupassen oder zumindest zu überprüfen.

Entsprechend beschreibt WO 2023/275158 A1 im Zusammenhang mit der Gewebedeformation eine Technik, die eine hohe Genauigkeit der Navigation während des gesamten chirurgischen Eingriffs sicherstellt, wenn Gewebe durch den Eingriff räumlich gesehen verändert wird oder sich bewegt.

Im Zusammenhang mit einer schleichenden Verschiebung des Patienten-Koordinatensystems gegenüber den mittels des Navigationssystems nachverfolgten Markern beschreibt beispielsweise WO 2023/110134 A1 eine Technik, bei der eine Form und die Position einer Kontur am Schädel des Patienten in Bezug auf Marker einer Kopfklemme bestimmt wird. Dann wird jede Abweichung von dieser Anfangsposition zu einem späteren Zeitpunkt auf eine unbeabsichtigte Bewegung des Schädelknochens innerhalb der immobilisierenden Kopfklemme gewertet. Eine solche Technik ist allerdings darauf angewiesen, dass die Kopfklemme selbst lagefest angeordnet ist. Manchmal kann es allerdings vorkommen, dass der Patient umgelagert werden soll und sich damit die Kopfklemme selbst im globalen Referenz-Koordinatensystem bewegt. In einem solchen Szenario ist die Abweichung von der Anfangsposition der Kontur am Schädel nicht unbeabsichtigt.

Ferner offenbart WO 2022/047572 A1 im Zusammenhang mit einer schleichenden Verschiebung des Patienten-Koordinatensystems Techniken, die eine intraoperative Bewertung der Registrierung in Verbindung mit einem nachverfolgbaren Referenz-Koordinatensystem ermöglichen. Zum Beispiel kann das Referenz-Koordinatensystem in Bezug auf das Objekt mit einer festen Position und Ausrichtung relativ zum Patienten gesichert werden, sodass Änderungen der Position und/oder Ausrichtung des Patienten in Änderungen der Position und/oder Ausrichtung des Referenz-Koordinate widergespiegelt werden. Es wird eine Ausgangsposition und Ausrichtung des nachverfolgbaren Referenz-Koordinatensystems bestimmt. Die Position und Ausrichtung des nachverfolgbaren Referenz-Koordinatensystems wird anschließend nachverfolgt und eine geschätzte aktualisierte Position eines Orientierungspunktes wird durch Aufrechterhaltung eines festen dreidimensionalen Versatzes zwischen dem Orientierungspunkt und dem nachverfolgbaren Referenz-Koordinatensystems bestimmt. Es wird dann die geschätzte aktualisierte Position des Orientierungspunkts angezeigt, z. B. in einem intraoperativen Bild. Der Benutzer kann einen Registrierungsfehler visuell beobachten, indem er eine fehlende räumliche Übereinstimmung zwischen dem tatsächlichen Standort des Orientierungspunkts und dem geschätzten aktualisierten Standort feststellt. Eine solche Technik weist den Nachteil auf, dass ein Benutzereingriff im Zusammenhang mit der Bewertung des Fehlers der Registrierung erforderlich ist. Generell ist es aber erstrebenswert, dass die kognitive Last für den Chirurgen für andere als die chirurgische Kernaufgabe während des chirurgischen Eingriffs minimiert wird.

### ZUSAMMENFASSUNG

Deshalb besteht ein Bedarf für verbesserte Techniken, um eine Schätzung einer Patienten-Registrierung anzupassen und/oder zu bewerten. Es besteht ein Bedarf für Techniken, um entsprechende Kontextinformation für die Schätzung der Patienten-Registrierung zu bestimmen, wobei die Kontextinformation z.B. eine Unsicherheit oder eine alternative Schätzung der Patienten-Registrierung angibt. Insbesondere besteht ein Bedarf um eine mittels künstlichen Marken, die an einer Klemme befestigt sind, geschätzte Patienten-Registrierung anzupassen und/oder zu bewerten. Ferner besteht ein Bedarf für Techniken, mittels derer ein schleichender Shift zwischen künstlichen Markern an einer Halterung für einen Patienten und dem Patienten-Koordinatensystem während des chirurgischen Eingriffs überwacht werden kann.

Diese Aufgabe wird gelöst von den Merkmalen der unabhängigen Patentansprüche. Die Merkmale der abhängigen Patentansprüche definieren Ausführungsformen.

Es wird ein chirurgisches Assistenzsystem offenbart. Das chirurgische Assistenzsystem umfasst mindestens einem Prozessor sowie mindestens einen Speicher. Dabei ist der mindestens einem Prozessor eingerichtet, um aus dem mindestens einen Speicher Programmcode zu laden und um diesen Programmcode auszuführen. Der mindestens eine Prozessor verfolgt, basierend auf dem Programmcode, eine Lage einer Halterung während eines Beobachtungszeitraums in einem Referenz-Koordinatensystem nach. Die Halterung ist dabei lagefest an einem Skelett eines Patienten angebracht. Das Nachverfolgen der Lage der Halterung basiert auf Referenzmarkern, die an der Halterung befestigt sind.

Der mindestens eine Prozessor bestimmt, basierend auf dem Ausführen des Programmcodes, eine Schätzung einer Patienten-Registrierung im Referenz-Koordinatensystem während des Beobachtungszeitraums. Die Schätzung der Patienten-Registrierung wird dabei basierend auf deren Nachverfolgung der Lage der Halterung bestimmt.

Der mindestens eine Prozessor erhält, basierend auf dem Ausführen des Programmcodes, intraoperativen Bilddaten. Die intraoperativen Bilddaten sind während des Beobachtungszeitraums mittels eines chirurgischen Visualisierungssystems erfasst. Das chirurgische Visualisierungssystem ist dabei in Bezug auf den Patienten beweglich.

Der mindestens eine Prozessor bestimmt, basierend auf dem Ausführen des Programmcodes, Kontextinformation für die Schätzung der Patienten-Registrierung; und erzeugt ferner ein Steuersignal basierend auf der Kontextinformation. Der Steuersignal ist für ein oder mehrere mit dem chirurgischen Assistenzsystem assoziierte Komponenten.

Es sind unterschiedliche Varianten offenbart, um die Kontextinformation zu bestimmen.

In einer Variante bestimmt der mindestens eine Prozessor, basierend auf dem Ausführen des Programmcodes sowie basierend auf den intraoperativen Bilddaten, eine Position und/oder eine Orientierung einer in den intraoperativen Bilddaten dargestellten anatomischen Landmarke. Diese anatomische Landmarke ist in Bezug auf das Skelett lagefest. Außerdem führt der mindestens eine Prozessor, basierend auf dem Ausführen des Programmcodes, einen Vergleich durch. Dieser Vergleich setzt eine Änderung der Schätzung der Patienten-Registrierung während des Beobachtungszeitraums in Bezug zu einer Änderung der Position und/oder Orientierung der anatomischen Landmarke während des Beobachtungszeitraums.

In einer anderen Variante (die mit der voranstehend beschriebenen Variante kombiniert werden kann oder auch in Isolation ausgeführt werden kann) bestimmt der mindestens eine Prozessor, basierend auf den intraoperativen Bilddaten sowie basierend auf dem Ausführen des Programmcodes, eine Schätzung einer Position und/oder eine Orientierung einer anatomischen Landmarke in dem Referenz-Koordinatensystem. Diese anatomische Landmarke ist in Bezug auf das Skelett lagefest. Außerdem führt der mindestens eine Prozessor, basierend auf dem Ausführen des Programmcodes, einen Vergleich der Schätzung der Position und/oder Orientierung der anatomischen Landmarke mit einer vorgegebenen Referenz-Position und/oder Referenz-Orientierung der anatomischen Landmarke aus. Die Referenz-Position und/oder die Referenz-Orientierung sind aus Referenz-Bilddaten des Patienten und anhand der Schätzung der Patienten-Registrierung bestimmt.

Es wären auch noch andere Varianten denkbar. Insbesondere ist es möglich, dass die Kontextinformation mit einer Technik bestimmt wird, die alternativ zur Technik ist, mit der die Schätzung der Patienten-Registrierung bestimmt wird. Das bedeutet, dass z.B. die Marker an der Halterung für das Bestimmen der Kontextinformation nicht (direkt) berücksichtigt werden.

Ein Computer-implementiertes Verfahren umfasst das Nachverfolgen einer Lage einer Halterung während eines Beobachtungszeitraums in einem Referenz-Koordinatensystem. Die Lage der Halterung wird basierend auf Referenzmarkern nachverfolgt. Die Referenzmarker sind lagefest an der Halterung befestigt. Die Halterung ist wiederum am Patienten angebracht. Das Verfahren umfasst auch das Bestimmen einer Schätzung einer Patienten-Registrierung im Referenz-Koordinatensystem während des Beobachtungszeitraums sowie basierend auf der Nachverfolgung der Lage der Halterung. Das Verfahren umfasst ferner das Erhalten von intraoperativen Bilddaten. Die intraoperativen Bilddaten sind während des Beobachtungszeitraums mittels eines chirurgischen Visualisierungssystems erfasst. Das chirurgische Visualisierungssystem ist in Bezug auf den Patienten beweglich. Das Verfahren umfasst auch das Bestimmen einer Position und/oder Orientierung einer anatomischen Landmarke. Die anatomische Landmarke ist in den intraoperativen Bilddaten dargestellt. Die anatomische Landmarke ist in Bezug auf das Skelett lagefest. Die Position und/oder Orientierung der anatomischen Landmarke wird dabei basierend auf den intraoperativen Bilddaten bestimmt. Außerdem umfasst das Verfahren das Durchführen eines Vergleichs. Dieser Vergleich setzt eine Änderung der Schätzung der Patienten-Registrierung während des Beobachtungszeitraums in Bezug zu einer Änderung der Position und/oder Orientierung der anatomischen Landmarke während des Beobachtungszeitraums. Das Verfahren umfasst ferner das Bestimmen von Kontextinformation für die Schätzung der Patienten-Registrierung basierend auf einem Ergebnis dieses Vergleichs, sowie, basierend auf der Kontextinformation, das Erzeugen eines Steuersignals für eine oder mehrere mit einem chirurgischen Assistenzsystem assoziierte Komponenten.

Eine elektronische Datenverarbeitungsvorrichtung umfasst mindestens einen Prozessor und mindestens einen Speicher. Der mindestens eine Prozessors ist dabei eingerichtet, um Programmcode aus dem mindestens einen Speicher zu laden und auszuführen. Der mindestens eine Prozessor führt basierend auf dem Ausführen des Programmcodes die folgenden Schritte aus: Erhalten von intraoperativen Bilddaten, welche während eines Beobachtungszeitraums mittels eines chirurgischen Visualisierungssystems erfasst sind. Das chirurgische Visualisierungssystems ist dabei in Bezug auf einen Patienten beweglich. Bestimmen einer Position und/oder Orientierung einer in den intraoperativen Bilddaten dargestellten anatomischen Landmarke, welche in Bezug auf das Skelett lagefest ist; Durchführen eines Vergleichs, welche eine Änderung einer Schätzung der Patienten-Registrierung während des Beobachtungszeitraums in Bezug setzt zu einer Änderung der Position und/oder Orientierung der anatomischen Landmarke während des Beobachtungszeitraums; basierend auf einem Ergebnis des Vergleichs, Bestimmen von Kontextinformatoin für die Schätzung der Patienten-Registrierung und basierend auf der Kontextinformation Erzeugen eines Steuersignals für ein oder mehrere mit dem chirurgischen Assistenzsystem assoziierte Komponenten.

Ein Computer-implementiertes Verfahren umfasst das Erhalten von intraoperativen Bilddaten, welche während eines Beobachtungszeitraums mittels eines chirurgischen Visualisierungssystems erfassten. Das chirurgische Visualisierungssystems dabei in Bezug auf einen Patienten beweglich. Das Verfahren umfasst ferner das Bestimmen einer Position und/oder Orientierung einer in den intraoperativen Bilddaten dargestellten anatomischen Landmarke. Dies basiert auf den intraoperativen Bilddaten. Die anatomische Landmarke ist in Bezug auf das Skelett lagefest. Das Verfahren umfasst ferner das Durchführen eines Vergleichs. Der Vergleich setzt eine Änderung einer Schätzung der Patienten-Registrierung während des Beobachtungszeitraums in Bezug zu einer Änderung der Position und/oder Orientierung der anatomischen Landmarke während des Beobachtungszeitraums. Außerdem umfasst das Verfahren das Bestimmen von Kontextinformation für die Schätzung der Patienten-Registrierung basierend auf einem Ergebnis des Vergleichs. Das Verfahren umfasst ferner das Erzeugen eines Steuersignals für ein oder mehrere mit dem chirurgischen Assistenzsystem assoziierte Komponenten basierend auf der Kontextinformation.

Ein Computer-implementiertes Verfahren umfasst das Nachverfolgen einer Lage einer Halterung während eines Beobachtungszeitraums in einem Referenz-Koordinatensystem. Das Nachverfolgen basiert dabei auf Referenzmarkern. Die Referenz Markers in lagefest an der Halterung befestigt. Die Halterung ist am Skelett eines Patienten angebracht. Das Verfahren umfasst ferner das Bestimmen einer Schätzung einer Patienten-Registrierung im Referenz-Koordinatensystem während des Beobachtungszeitraums basierend auf der Nachverfolgung der Lage der Halterung. Außerdem umfasst das Verfahren das Erhalten von intraoperativen Bilddaten. Die intraoperativen Bilddaten sind während des Beobachtungszeitraums mittels eines chirurgischen Visualisierungssystems erfasst. Das chirurgische Visualisierungssystem ist in Bezug auf den Patienten beweglich. Außerdem umfasst das Verfahren das Bestimmen einer Schätzung einer Position und/oder Orientierung einer anatomischen Landmarke, die in den intraoperativen Bilddaten dargestellt ist. Diese Schätzung wird basierend auf den intraoperativen Bilddaten bestimmt. Die Schätzung wird im Referenz-Koordinatensystem bestimmt. Die anatomische Landmarke ist in Bezug auf das Skelett lagefest. Außerdem umfasst das Verfahren das Durchführen eines Vergleichs der Schätzung der Position und/oder der Orientierung der anatomischen Landmarke mit einer vorgegebenen Referenz-Position und/oder Referenz-Orientierung, welche aus Referenz-Bilddaten des Patienten und anhand der Schätzung der Patientin-Registrierung bestimmt ist. Das Verfahren umfasst ferner das Bestimmen von Kontextinformation für die Schätzung der Patientin-Registrierung basierend auf einem Ergebnis des Vergleichs sowie, basierend auf der Kontextinformation, erzeugen eines Steuersignals für ein oder mehrere mit einem chirurgischen Assistenzsystem assoziierte Komponenten.

Eine elektronische Datenverarbeitungsvorrichtung umfasst mindestens einem Prozessor mindestens einen Speicher. Der mindestens eine Prozessor ist eingerichtet, um Programmcode aus dem mindestens einen Speicher zu laden und auszuführen. Der mindestens eine Prozessor führt basierend auf dem Ausführen des Programmcodes die folgenden Schritte aus: Erhalten von intraoperativen Bilddaten, welche während eines Beobachtungszeitraums mittels eines chirurgischen Visualisierungssystems erfasst sind, wobei das chirurgische Visualisierungssystem in Bezug auf einen Patienten beweglich ist; basierend auf den intraoperativen Bilddaten, Bestimmen einer Schätzung einer Position und/oder Orientierung einer in den intraoperativen Bilddaten dargestellten anatomischen Landmarken einem Referenz-Koordinatensystem, wobei die anatomische Landmarken Bezug auf das Skelett lagefest ist; Durchführen eines Vergleichs der Schätzung der Position und/oder Orientierung der anatomischen Landmarke mit einer vorgegebenen Referenz-Position und/oder Referenz-Orientierung, die aus Referenz-Bilddaten des Patienten und anhand einer Schätzung der Patienten-Registrierung bestimmt ist; basierend auf einem Ergebnis des Vergleichs, Bestimmen von Kontextinformation für die Schätzung der Patientenregistrierung, und basierend auf der Kontextinformation, Erzeugen eines Steuersignals für ein oder mehrere mit dem chirurgischen Assistenzsystem assoziierte Komponenten.

Ein Computer-implementiertes Verfahren umfasst die folgenden Schritte: Erhalten von intraoperativen Bilddaten, welche während eines Beobachtungszeitraums mittels eines chirurgischen Visualisierungssystems erfasst sind, wobei das chirurgische Visualisierungssystem in Bezug auf einen Patienten beweglich ist; basierend auf den intraoperativen Bilddaten, Bestimmen einer Schätzung einer Position und/oder Orientierung einer in den intraoperativen Bilddaten dargestellten anatomischen Landmarken einem Referenz-Koordinatensystem, wobei die anatomische Landmarken Bezug auf das Skelett lagefest ist; Durchführen eines Vergleichs der Schätzung der Position und/oder Orientierung der anatomischen Landmarke mit einer vorgegebenen Referenz-Position und/oder Referenz-Orientierung, die aus Referenz-Bilddaten des Patienten und anhand einer Schätzung der Patienten-Registrierung bestimmt ist; basierend auf einem Ergebnis des Vergleichs, Bestimmen von Kontextinformation für die Schätzung der Patientenregistrierung, und basierend auf der Kontextinformation, Erzeugen eines Steuersignals für ein oder mehrere mit dem chirurgischen Assistenzsystem assoziierte Komponenten.

Eine elektronische Datenverarbeitungsvorrichtung für ein chirurgisches Navigationssystem umfasst mindestens einem Prozessor sowie mindestens einen Speicher. Der mindestens eine Prozessors dabei eingerichtet, um Programmcode aus dem mindestens einen Speicher zu laden und auszuführen. Der mindestens eine Prozessor führt basierend auf dem Ausführen des Programmcodes folgende Schritte aus: basierend auf Referenzmarkern, die lagefest an einer an einem Skelett eines Patienten angebrachten Halterung befestigt sind, Nachverfolgen einer Lage der Halterung während eines Beobachtungszeitraums in einem Referenz-Koordinatensystem sowie Bestimmen einer Schätzung einer Patienten-Registrierung im Referenz-Koordinatensystem während des Beobachtungszeitraums basierend auf der Nachverfolgung der Lage der Halterung; sowie Empfangen eines Steuersignals, welches Kontextinformation für die Schätzung der Patienten-Registrierung indiziert (wobei diese Kontextinformation gemäß verschiedenen hierin offenbarten Varianten bestimmt sein kann); sowie basierend auf der Kontextinformation, Anpassen der Schätzung der Patienten-Registrierung.

Ein Computer-implementiertes Verfahren umfasst: basierend auf Referenzmarkern, die lagefest an einer an einem Skelett eines Patienten angebrachten Halterung befestigt sind, Nachverfolgen einer Lage der Halterung während eines Beobachtungszeitraums in einem Referenz-Koordinatensystem sowie Bestimmen einer Schätzung einer Patienten-Registrierung im Referenz-Koordinatensystem während des Beobachtungszeitraums basierend auf der Nachverfolgung der Lage der Halterung; sowie Empfangen eines Steuersignals, welches Kontextinformation für die Schätzung der Patienten-Registrierung indiziert (wobei diese Kontextinformation gemäß verschiedenen hierin offenbarten Varianten bestimmt sein kann); sowie basierend auf der Kontextinformation, Anpassen der Schätzung der Patienten-Registrierung.

Ein chirurgisches Assistenzsystem umfasst mindestens einen Prozessor und mindestens einen Speicher, wobei der mindestens eine Prozessor eingerichtet ist, um Programmcode aus dem mindestens einen Speicher zu laden und auszuführen, wobei der mindestens eine Prozessor basierend auf dem Ausführen des Programmcodes die folgenden Schritte ausführt: basierend auf Referenzmarkern, die lagefest an einer an einem Skelett eines Patienten angebrachten Halterung befestigt sind: Nachverfolgen einer Lage der Halterung während eines Beobachtungszeitraums in einem Referenz-Koordinatensystem; und basierend auf der Nachverfolgung der Lage der Halterung, Bestimmen einer Schätzung einer Patienten-Registrierung im Referenz-Koordinatensystem während des Beobachtungszeitraums; und Erhalten von intraoperativen Bilddaten, welche während des Beobachtungszeitraums mittels eines chirurgischen Visualisierungssystems erfasst sind, wobei das chirurgische Visualisierungssystem in Bezug auf den Patienten beweglich ist; und basierend auf den intraoperativen Bilddaten, Bestimmen einer Position und/oder Orientierung einer in den intraoperativen Bilddaten dargestellten anatomischen Landmarke, wobei die anatomische Landmarke in Bezug auf das Skelett lagefest ist; und Durchführen eines Vergleichs der Position und/oder Orientierung der anatomischen Landmarke mit einer vorgegebenen Referenz-Position und/oder Referenz-Orientierung, die aus Referenz-Bilddaten des Patienten und anhand der Schätzung der Patienten-Registrierung bestimmt ist; und basierend auf einem Ergebnis des Vergleichs, Bestimmen von Kontextinformation für die Schätzung der Patienten-Registrierung; und basierend auf der Kontextinformation, Erzeugen eines Steuersignals für ein oder mehrere mit dem chirurgischen Assistenzsystem assoziierte Komponenten.

Ein Computer-implementiertes Verfahren umfasst: basierend auf Referenzmarkern, die lagefest an einer an einem Skelett eines Patienten angebrachten Halterung befestigt sind: Nachverfolgen einer Lage der Halterung während eines Beobachtungszeitraums in einem Referenz-Koordinatensystem; und basierend auf der Nachverfolgung der Lage der Halterung, Bestimmen einer Schätzung einer Patienten-Registrierung im Referenz-Koordinatensystem während des Beobachtungszeitraums; und Erhalten von intraoperativen Bilddaten, welche während des Beobachtungszeitraums mittels eines chirurgischen Visualisierungssystems erfasst sind, wobei das chirurgische Visualisierungssystem in Bezug auf den Patienten beweglich ist; und basierend auf den intraoperativen Bilddaten, Bestimmen einer Position und/oder Orientierung einer in den intraoperativen Bilddaten dargestellten anatomischen Landmarke, wobei die anatomische Landmarke in Bezug auf das Skelett lagefest ist; und Durchführen eines Vergleichs der Position und/oder Orientierung der anatomischen Landmarke mit einer vorgegebenen Referenz-Position und/oder Referenz-Orientierung, die aus Referenz-Bilddaten des Patienten und anhand der Schätzung der Patienten-Registrierung bestimmt ist; und basierend auf einem Ergebnis des Vergleichs, Bestimmen von Kontextinformation für die Schätzung der Patienten-Registrierung; und basierend auf der Kontextinformation, Erzeugen eines Steuersignals für ein oder mehrere mit dem chirurgischen Assistenzsystem assoziierte Komponenten.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen. Beispielsweise können Techniken, die im Zusammenhang mit einer Variante zur Bestimmung der Kontextinformation beschrieben sind, auch für andere Varianten zur Bestimmung der Kontextinformation verwendet werden.

### KURZE BESCHREIBUNG DER FIGUREN

FIG. 1 illustriert schematisch ein System mit einem Operationsmikroskop und einem Navigationssystem gemäß verschiedenen Beispielen.
FIG. 2 illustriert schematisch mehrere Koordinatensysteme für das System aus FIG. 1 und entsprechende Transformationen bzw. Registrierungen zwischen den Koordinatensystemen.
FIG. 3 ist ein Flussdiagramm eines Verfahrens gemäß verschiedenen Beispielen, wobei das Verfahren aus FIG. 3 der initialen Bestimmung einer Patienten-Registrierung dient.
FIG. 4 ist ein Flussdiagramm gemäß verschiedenen Beispielen, wobei das Verfahren aus FIG. 4 der Bestimmung einer Schätzung einer Patienten-Registrierung sowie der Bestimmung von Kontextinformation für die Schätzung der Patienten-Registrierung dient.
FIG. 5 illustriert schematisch einen Vergleich zwischen der Schätzung einer Position und/oder Orientierung einer starren anatomischen Landmarke mit einer vorgegebenen Referenz-Position und/oder Referenz-Orientierung gemäß verschiedenen Beispielen.
FIG. 6 illustriert schematisch einen Vergleich, der eine Änderung der Schätzung der Patienten-Registrierung in Bezug setzt zu einer Änderung der Position und/oder Orientierung einer anatomischen Landmarke während eines Beobachtungszeitraums und gemäß verschiedenen Beispielen.
FIG. 7 ist ein Flussdiagramm eines Verfahrens gemäß verschiedenen Beispielen, wobei das Verfahren aus FIG. 7 die Auswertung von intraoperativen Bilddaten zum Bestimmen von Kontextinformation für eine Schätzung einer Patienten-Registrierung betrifft.
FIG. 8 ist ein Flussdiagramm eines Verfahrens gemäß verschiedenen Beispielen, wobei das Verfahren aus FIG. 8 die Nachverfolgung einer in intraoperativen Bilddaten dargestellten anatomischen Landmarke anhand einer Auswertung der intraoperativen Bilddaten betrifft.
FIG. 9 illustriert schematisch die teilweise Sichtbarkeit einer anatomischen Landmarke in einer Zeitsequenz von intraoperativen Bildern gemäß verschiedenen Beispielen.
FIG. 10 illustriert schematisch eine elektronische Datenverarbeitungsvorrichtung für ein chirurgisches Assistenzsystem gemäß verschiedenen Beispielen.

### DETAILLIERTE BESCHREIBUNG

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Nachfolgend wird die vorliegende Erfindung anhand bevorzugter Ausführungsformen unter Bezugnahme auf die Zeichnungen näher erläutert. In den Figuren bezeichnen gleiche Bezugszeichen gleiche oder ähnliche Elemente. Die Figuren sind schematische Repräsentationen verschiedener Ausführungsformen der Erfindung. In den Figuren dargestellte Elemente sind nicht notwendigerweise maßstabsgetreu dargestellt. Vielmehr sind die verschiedenen in den Figuren dargestellten Elemente derart wiedergegeben, dass ihre Funktion und genereller Zweck dem Fachmann verständlich wird. In den Figuren dargestellte Verbindungen und Kopplungen zwischen funktionellen Einheiten und Elementen können auch als indirekte Verbindung oder Kopplung implementiert werden. Eine Verbindung oder Kopplung kann drahtgebunden oder drahtlos implementiert sein. Funktionale Einheiten können als Hardware, Software oder eine Kombination aus Hardware und Software implementiert werden.

Nachfolgend werden Techniken offenbart, bei denen eine Schätzung einer Patienten-Registrierung in einem globalen Referenz-Koordinatensystems mittels Nachverfolgung von Markern erfolgt, die nicht direkt am Patienten befestigt sind. Die Marker werden von einem Navigationssystem verfolgt. Die Marker sind an einer Halterung, die lösbar am Patienten angebracht ist, befestigt. Die Halterung kann zum Beispiel eine Klemme sein. Die Halterung kann derart am Patienten befestigt sein, dass eine Relativbewegung zwischen der Halterung einerseits und dem Skelett des Patienten andererseits möglich ist.

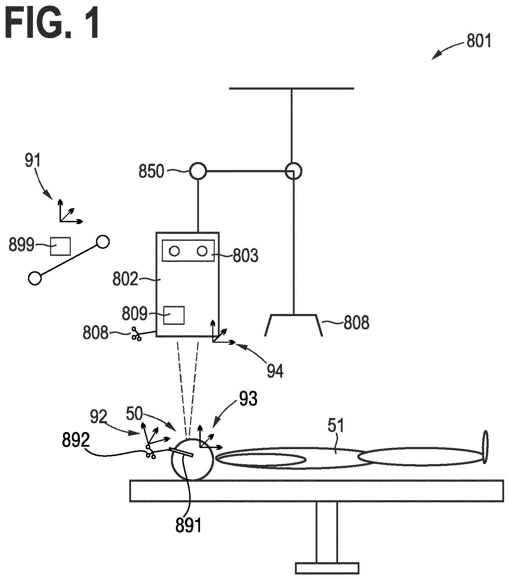

Die Lage der Marker wird im globalen Referenz-Koordinatensystem durch das Navigationssystem direkt und absolut und wiederholt gemessen. Basierend auf der entsprechenden Nachverfolgung der Lage der Marker über der Zeit kann eine Anpassung der Schätzung der Patienten-Registrierung ausgehend von einer initialen Patienten-Registrierung erfolgen. Diese und weitere Zusammenhänge sind in FIG. 1 illustriert.

FIG. 1 illustriert schematisch ein chirurgisches System 801. Das chirurgische System 801 umfasst ein Operationsmikroskop 802, welches im Beispiel der FIG. 1 sowohl mit einem Okular 803 (optional) wie auch mit einem digitalen optischen Kanal 809 zum Erfassen von intraoperativen Bilddaten ausgestattet ist.

Das Operationsmikroskop 802 ist an einem Stativ 850 befestigt, so dass die Pose, mit der eine Eingriffsregion 50 eines Patienten 51 abgebildet wird (gestrichelte Linien in FIG. 1) verändert werden kann. Das Operationsmikroskop 802 kann also bewegt werden. Ein entsprechendes Mikroskop-Koordinatensystem 94, welches sich zusammen mit dem Operationsmikroskop 802 in einem globalen Referenz-Koordinatensystem 91 bewegt, ist dargestellt. Das globale Referenz-Koordinatensystem ist durch ein Navigationssystem 899 aufgespannt bzw. definiert.

Um die Lage des Operationsmikroskops 802 im globalen Referenz-Koordinatensystem 91 zu bestimmen, sind künstliche Marker 808 am Operationsmikroskop 802 befestigt. Diese künstlichen Marker 808 werden vom Navigationssystem 899, welches ein oder mehrere entsprechende Sensoren aufweist, erkannt und deren Lage anschließend nachverfolgt. Die sechsdimensionale (6-D) Lage des Operationsmikroskops 802 im globalen Koordinatensystem 91 kann derart bestimmt werden. Es kann also die Position und die Orientierung des Operationsmikroskops 802 bestimmt werden. Das lokale Mikroskop-Koordinatensystem 94 kann also mit dem globalen Referenz-Koordinatensystem 91 registriert werden. Zusammen mit einem Kameramodell des Operationsmikroskops 802, welches die Abbildung einer Objektebene in die Bildebene der intraoperativen Bilddaten beschreibt, kann dann die Lage der Objektebene im globalen Referenz-Koordinatensystem 91 ermittelt werden.

Dabei wird angemerkt, dass die Bestimmung der Lage des Operationsmikroskops 802 nicht nur mit Markern 808 stattfinden kann. Es wäre zum Beispiel alternativ oder zusätzlich denkbar, dass Positionierdaten zur Einstellung des Stativ 850 an das Navigationssystem 899 übermittelt werden. Grundsätzlich sind Verfahren zur Bestimmung der Lage der Objektebene des Operationsmikroskops nicht entscheidend für die hierin beschriebenen Techniken; vielmehr können die hierin beschriebenen Techniken auf verschiedenste vorbekannte Verfahren zurückgreifen.

Dargestellt sind in FIG. 1 auch ein lokales Patienten-Koordinatensystem 93. Die Lage bestimmter anatomischer Merkmale, die für den chirurgischen Eingriff wichtig sind, sind im lokalen Patienten-Koordinatensystem definiert. Die Lage solcher anatomischen Merkmale ist auch in präoperativen Volumenbilddaten bekannt.

Um die präoperativen Volumenbilddaten geeignet mit den intraoperativ mittels des Operationsmikroskops 802 erfassten Bilddaten zu überlagern, wird das lokale Patienten-Koordinatensystem 93 mit dem globalen Referenz-Koordinatensystem 91 registriert. Anhand der Kenntnis der Patienten-Registrierung können präoperative Bilddaten (virtuell) jeweils so im globalen Referenz-Koordinatensystem 91 positioniert werden, dass die präoperativen Bilddaten mit den in den intraoperativen Bilddaten überlappen.

Dazu sind Techniken bekannt, um eine initiale Messung der Oberflächentopographie des Schädels im Bereich der Eingriffsregion 50 durchzuführen, um derart die Patienten-Registrierung initial direkt und absolut zu bestimmen. Typischerweise kann eine solche initiale Messung (zum Beispiel unter Verwendung eines Pointers oder mittels eines Lasers) nicht während des gesamten chirurgischen Eingriffs wiederholt werden. Deshalb muss in einem Beobachtungszeitraum, der während des chirurgischen Eingriffs stattfindet, auf eine Schätzung der Patienten-Registrierung zurückgegriffen werden. Zur Bestimmung der Schätzung der Patienten-Registrierung ist am Patienten 51 im Bereich der Eingriffsregion 50 eine Halterung 891 befestigt, z.B. eine Mayfield-Klemme. An der Halterung 891 sind Marker 892 angebracht, welche eine Nachverfolgung der Lage der Halterung 891 im globalen Referenz-Koordinatensystem 91 durch das Navigationssystem 899 ermöglicht. Wenn die Klemme 891 lagefest am Patienten 51 angebracht ist, wird jede Lageänderung des Marker-Koordinatensystems 92 begleitet von derselben Lageänderung des Patienten-Koordinatensystems 93. Es kann also eine relative Schätzung der Patienten-Registrierung während des Beobachtungszeitraums erfolgen.

Allerdings kann es aufgrund von schleichenden Shifts vorkommen, dass sich die Lage des Marker-Koordinatensystems 92 gegenüber der Lage des Patienten-Koordinatensystems 93 im Laufe des chirurgischen Eingriffs verändert. Dann akkumulieren sich entsprechende systematische Fehler der relativen Schätzung der Patienten-Registrierung. Damit ist die Patienten-Registrierung verfälscht und entsprechend kann auch keine genaue Überlagerung der intraoperativen Bilddaten mit den präoperativen Bilddaten erfolgen.

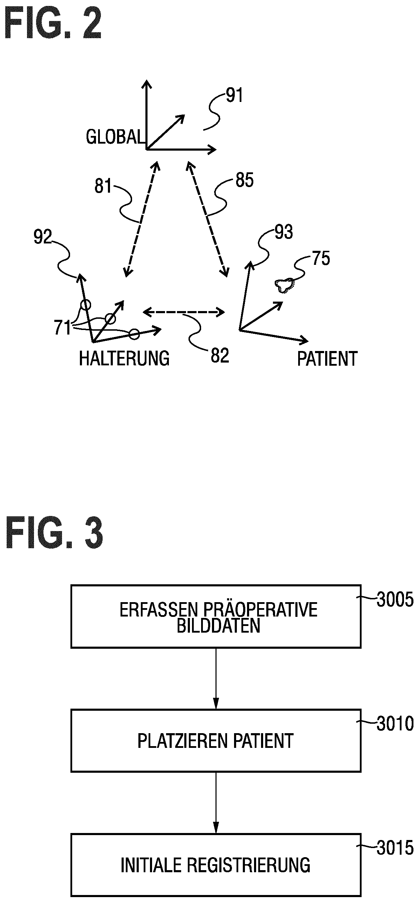

FIG. 2 illustriert schematisch die Patienten-Registrierung 85. Die Patienten-Registrierung 85 beschreibt eine Transformation zwischen dem lokalen Patienten-Koordinatensystem 93 und dem globalen Referenz-Koordinatensystem 91.

Außerdem ist in FIG. 2 dargestellt, dass das lokale Patienten-Koordinatensystem 93 über eine Registrierung 82 mit dem Marker-Koordinatensystem 92 in Beziehung steht. Das Marker-Koordinatensystem 92 steht über wiederrum eine Registrierung 81 mit dem globalen Koordinatensystem 91 in Beziehung. Die Registrierung 81 kann mittels des Navigationssystems 899 während eines längeren Beobachtungszeitraums oder sogar während des gesamten chirurgischen Eingriffs direkt bestimmt werden, weil die Lage der Halterung 891 anhand entsprechender Marker direkt gemessen werden kann.

Das Marker-Koordinatensystem 92 dient als Hilfskoordinatensystem / Proxy, um eine Schätzung der Patienten-Registrierung 85 ausgehend von ihrer initialen Bestimmung der Patienten-Registrierung zu bestimmen. Änderungen der Lage des Marker-Koordinatensystems 92 in Bezug auf das globale Referenz-Koordinatensystem 91 werden bei dieser Schätzung auf Änderungen der Patienten-Registrierung 85 übertragen. In anderen Worten: ändert sich die Registrierung 81, so wird auch die Patienten-Registrierung 85 entsprechend geändert.

Um etwaige systematische und über der Zeit akkumulierende Fehler aufgrund einer Relativbewegung des Marker-Koordinatensystems 92 gegenüber dem Patienten-Koordinatensystem 93 zu erkennen oder sogar zu kompensieren, wird Kontextinformation ermittelt, wobei die Kontextinformation mit der Schätzung der Patienten-Registrierung assoziiert ist bzw. für diese ermittelt wird (wobei die Schätzung der Patienten-Registrierung wiederum auf Grundlage der Nachverfolgung der an der Halterung befestigten Marker bestimmt ist). Die Kontextinformation wird dann im Rahmen eines Steuersignals für die Steuerung von ein oder mehrere Komponenten des chirurgischen Systems 801 und/oder von ein oder mehreren anderen Komponenten, die mit dem chirurgischen System 801 assoziiert sind, verwendet.

Das Steuersignal wird dazu basierend auf der Kontextinformation bestimmt. Dies kann auf unterschiedliche Arten und Weisen geschehen. Beispielsweise könnte die Kontextinformation in einen Datenrahmen eingefügt werden, der dann das Steuersignal bildet. Neben einer solchen "Verpackung" der Kontextinformationen in einen Datenrahmen könnte aber auch eine Konvertierung der Kontextinformation erfolgen, beispielsweise um eine bestimmte Anforderung an die Datenstruktur der empfangenden Komponente zu erfüllen. Mittels des Steuersignals könnte beispielsweise eine Benutzerschnittstelle angesteuert werden, um bestimmte mit der Kontextinformation Hinweise an den Benutzer auszugeben. Mittels des Steuersignals kann also in manchen Beispielen eine Hinweisfunktion oder eine Warnfunktion erfüllt werden, beispielsweise wenn erkannt wird, dass der Fehler bzw. die Ungenauigkeit der Patientin-Registrierung 85 einem bestimmten Schwellenwert überschritten hat. Mittels des Steuersignals kann aber nicht nur eine Hinweisfunktion oder eine Warnfunktion erfüllt werden. Beispielweise wäre es in anderen Varianten denkbar, dass das Steuersignal an das Navigationssystem 899 übergeben wird und dann eine Anpassung der Patienten-Registrierung 85 basierend auf dem Steuersignal erfolgt wird. Das Steuersignal kann also für die Anforderung einer Korrektur der Anpassung der Patienten-Registrierung 85 verwendet werden.

Aus obenstehendem ist ersichtlich, dass mittels des Steuersignals unterschiedliche Funktionalitäten erfüllt werden können. Entsprechend dem breiten Spektrum an denkbaren Anwendungsszenarien für das Steuersignal kann auch die Kontextinformation unterschiedliche Ausbildungen annehmen. Einige beispielhafte Varianten für die Implementierung der Kontextinformation werden nachfolgend erläutert.

Die Kontextinformation kann zum Beispiel eine alternative Schätzung für die Patienten-Registrierung bereitstellen. Beispielsweise könnte die Kontextinformation bestimmt werden und dann im Rahmen des Steuersignals ggf. an das Navigationssystem 899 übergeben werden, welches dann (beispielsweise auf Anforderung des Steuersignals hin) eine Fusion der beiden alternativen Schätzungen für die Patienten-Registrierung durchführen kann. Mittels der Kontextinformation könnte also zum Beispiel eine Anpassung der anhand der Nachverfolgung der Lage der Marker bestimmten Schätzung der Patienten-Registrierung erfolgen.

Es wäre möglich, dass die Kontextinformation indikativ für eine Unsicherheit der Schätzung der Patienten-Registrierung ist. Damit kann beispielsweise eine Benutzerschnittstelle auf Grundlage der Kontextinformation und mittels des Steuersignals angesteuert werden, um eine entsprechende Warnung bei einer erhöhten Unsicherheit der Schätzung der Patienten-Registrierung an den Benutzer auszugeben. In einem solchen Fall wird die vom Navigationssystem anhand der Nachverfolgung der Lage der Marker 892 bestimmte Schätzung der Patienten-Registrierung 85 nicht verändert und besteht fort; die Überlagerung der intraoperativen Bilddaten mit den präoperativen Bilddaten erfolgt immer noch auf der gegebenenfalls mit einer großen Unsicherheit oder mit einem signifikanten Fehler behafteten Schätzung der Patienten-Registrierung 85. Es kann aber mittels des Steuersignals eine entsprechende Warnung an den Benutzer ausgegeben werden, so dass der Benutzer für diese Ungenauigkeit sensibilisiert wird.

Die Kontextinformation wird typischerweise nicht anhand der an der Halterung befestigten Marker bestimmt. Die Kontextinformation wird vielmehr mittels einer alternativen Modalität bestimmt. In verschiedenen hierin offenbarten Beispielen wird die Kontextinformation basierend auf intraoperativen Bilddaten, die mittels eines Operationsmikroskops erfasst werden und die den Patienten bzw. insbesondere eine Eingriffsregion des Patienten abbilden, bestimmt.

Die intraoperativen Bilddaten können zum Beispiel eine Zeitsequenz von zweidimensionalen (2-D) Bildern, gegebenenfalls mit stereoskopische Tiefeninformation, beinhalten. Die intraoperativen Bilddaten können in einem Videoformat vorliegen.

Die intraoperativen Bilddaten können analysiert und ausgewertet werden, um ein oder mehrere anatomische Landmarken aufzufinden. Die Position und/oder Orientierung dieser ein oder mehreren anatomischen Landmarken in der Bildebene oder einem anderen Koordinatensystem kann dann bestimmt werden und mit einer örtlichen oder zeitlichen Referenz verglichen werden.

So ist in FIG. 2 eine anatomisches Landmarke 75 dargestellt, die eine im lokalen Patienten-Koordinatensystem 93 definierte Lage aufweist. Mittels der Patienten-Registrierung 85 kann die Position des anatomischen Merkmals in den intraoperativen Bilddaten bestimmt werden, weil die Lage der anatomischen Landmarke 75 in Bezug auf eine Objektebene des Operationsmikroskops bestimmt werden kann. Weil die Position der anatomischen Landmarke in den präoperativen Bilddaten auch bekannt ist (gepunktete Linie), können die präoperativen Bilddaten überlagert mit den intraoperativen Bilddaten dargestellt werden.

Zur Bestimmung der Kontextinformation werden in den verschiedenen hierin beschriebenen Beispielen anatomische Landmarken, die in Bezug auf das Skelett des Patienten (beispielsweise in Bezug auf einen Referenzpunkt am Schädel) lagefest sind, verwendet. Beispiele sind zum Beispiel Knochen, Nervus Opticus, Falx Cerebri, Tentorium, Ateria Carotis. Solche lagefesten anatomischen Landmarken erlauben es insbesondere einen schleichenden Shift zwischen dem Marker-Koordinatensystem 92 und dem Patienten-Koordinatensystem 93 zu bestimmen - ohne weitere Verfälschung durch eine Deformation der Landmarken selber, wie es zum Beispiel beim Brainshift vorkommt.

Zwei Ansätze zur Bestimmung der Kontextinformation sind nachfolgend in TAB. 1 kurz zusammengefasst. Bei beiden Ansätzen wird eine bestimmte Position und/oder Orientierung einer anatomischen Landmarke verglichen mit einer Referenz. Die beiden Ansätze unterscheiden sich jedoch im verwendeten Typ der Referenz. Das ist in TAB. 1 aufgeschlüsselt.

**TAB. 1: Beispiele für das Bestimmen von Kontextinformation für eine indirekte, relative Schätzung einer Patienten-Registrierung anhand der Nachverfolgung der Lage der Marker.**

| | | DETAILS |
|---|---|---|
| 1 | Örtliche Referenz (cf. FIG. 5) | In dieser Technik wird die Position und/oder Orientierung der anatomischen Landmarke beispielsweise in dem globalen Referenz-Koordinatensystem geschätzt (beispielsweise anhand von intraoperativen Bilddaten, in denen die anatomische Landmarke lokalisiert wird) und dann diese geschätzte Position und/oder Orientierung verglichen mit einer Referenz-Position und/oder Referenz-Orientierung. Die Referenz-Position und/oder Referenz-Orientierung kann aus präoperativen Volumenbilddaten des Patienten anhand der derzeit gültigen Schätzung für die Patienten-Registrierung abgeleitet werden. Ergibt ein entsprechender Vergleich, dass eine Verschiebung zwischen der geschätzten Position und/oder Orientierung und der Referenz-Position und/oder Referenz-Orientierung vorliegt, so kann dies auf einen Fehler in der Marker-basierten indirekten Schätzung der Patienten-Registrierung zurückgeführt werden. |
| | | Es kann dann möglich sein, im Rahmen der Kontextinformation eine neue ("a-posteriori") Schätzung der Patienten-Registrierung zu bestimmen oder zumindest die Unsicherheit der Schätzung der Patienten-Registrierung zu ermitteln. |
| | | Anstatt einer Schätzung der Position und/oder Orientierung in dem globalen Referenz-Koordinatensystem könnte die Position und/oder Orientierung der anatomischen Landmarke auch in der Bildebene (dann jeweils 2-D bestimmt) mit einer entsprechenden Referenz verglichen werden. |
| 2 | Zeitliche Referenz (cf. FIG. 6) | In einer weiteren Technik wird - wiederum beispielsweise basierend auf intraoperativen Bilddaten - die Position und/oder Orientierung der anatomischen Landmarke in einem beliebigen Koordinatensystem bestimmt (weil keine örtliche, sondern eine zeitliche Referenz verwendet wird). Beispielsweise könnte die Position und/oder Orientierung der Landmarke direkt im lokalen Patienten-Koordinatensystem oder einem mit dem Operationsmikroskop assoziierten Referenz-Koordinatensystem bestimmt werden, etwa einem globalen Referenz-Koordinatensystem wie dem Referenz-Koordinatensystem 91, welches obenstehend diskutiert wurde. Dann kann ein Vergleich durchgeführt werden, der eine Änderung der Schätzung der Patienten-Registrierung während des Beobachtungszeitraums in Bezug setzt zu einer Änderung der Position und/oder Orientierung der anatomischen Landmarke während des Beobachtungszeitraums. |
| | | Dieser Vergleich kann Bewegungen des Marker-Koordinatensystems 92 gegenüber dem Patienten-Koordinatensystem 93 während des Beobachtungszeitraums indizieren. Beispielsweise kann sich die Halterung anders bewegen als der Patient. |
| | | Dies drückt sich in einer größeren Unsicherheit für die anhand der Registrierung 81 bestimmten Schätzung für die Patienten-Registrierung 85 aus. Es wäre auch möglich, eine neue Schätzung der Patienten-Registrierung zu bestimmen, ausgehend von der initialen Bestimmung der Patienten-Registrierung 85 und unter Verwendung der beobachteten Änderung der Position und/oder Orientierung der anatomischen Landmarke während des Beobachtungszeitraums. |

Details zu beiden in TAB. 1 beschriebenen Techniken werden später beschrieben. Beide in TAB. 1 beschriebenen Techniken bauen auf der initialen Bestimmung der Patienten-Registrierung und der anschließenden relativen Schätzung basierend auf der Nachverfolgung der Lage der Marker auf. In FIG. 3 werden zunächst Aspekte im Zusammenhang mit dieser initialen Bestimmung der Patienten-Registrierung erläutert.

FIG. 3 ist ein Flussdiagramm gemäß verschiedenen Beispielen. FIG. 3 illustriert Aspekte im Zusammenhang mit der initialen Patientenregistrierung, das heißt der initialen, absoluten Bestimmung der Patienten-Registrierung zwischen dem lokalen Patienten-Koordinatensystem einerseits und dem globalen Referenz-Koordinatensystem andererseits.

In Box 3005 werden präoperative Bilddaten erfasst. Die präoperativen Bilddaten können Volumenbilddaten sein. Beispielsweise können Magnetresonanz-Tomografie-Bilddaten oder Computertomografie-Bilddaten erfasst werden. Die präoperativen Bilddaten werden vor dem eigentlichen chirurgischen Eingriff erfasst.

In Box 3010 wird der Patient für den chirurgischen Eingriff vorbereitet. Box 3010 beinhaltet, dass der Patient in einen Operationsraum gebracht wird und auf einer Patientenliege angeordnet wird.

Der Operationsraum wird von einem Navigationssystem überwacht. Das Navigationssystem spannt ein globales Koordinatensystem auf (vergleiche FIG. 1: globales Referenz-Koordinatensystem 91).

Der Patient wird in Box 3015 im globalen Referenz-Koordinatensystem registriert. Die Patienten-Registrierung 85 wird direkt bestimmt. Beispielsweise kann eine Hautoberfläche im Bereich der Eingriffsregion vermessen werden; dann ist die Lage des lokalen Patienten-Koordinatensystems 93 im globalen Referenz-Koordinatensystem 91 zu Beginn des chirurgischen Eingriffs bekannt.

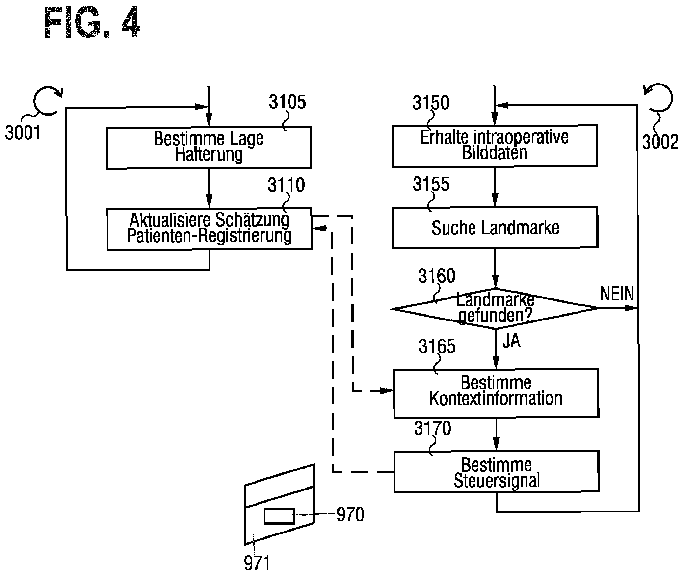

Die derart initial bestimmte Patienten-Registrierung wird nachfolgend angepasst. Dies erfolgt über das Hilfsmittel der am Patienten befestigten Halterung, die wiederum Marker aufweist, die vom Navigationssystem nachverfolgt werden. Das bedeutet also, dass eine Schätzung der Patienten-Registrierung bestimmt wird. Die Schätzung der Patienten-Registrierung wird basierend auf Änderungen der Lage der Marker und der initialen Patienten-Registrierung bestimmt. Entsprechende Aspekte sind in FIG. 4 dargestellt.

FIG. 4 ist ein Flussdiagramm eines beispielhaften Verfahrens. Das Verfahren aus FIG. 4 kann von einem chirurgischen Assistenzsystem ausgeführt werden. Das chirurgische Assistenzsystem kann ein oder mehrere elektronische Datenverarbeitungsvorrichtungen ausführen. Beispielsweise könnte das chirurgische Assistenzsystem eine elektronische Datenverarbeitungsvorrichtung eines Navigationssystems sowie/oder eine elektronische Datenverarbeitungsvorrichtung eines Operationsmikroskops umfassen. Unterschiedliche Teile des Verfahrens aus FIG. 4 können dann von unterschiedlichen elektronischen Datenverarbeitungsvorrichtungen ausgeführt werden. Es wäre aber auch denkbar, dass alle Schritte aus FIG. 4 von einer einzigen elektronischen Datenverarbeitungsvorrichtung ausgeführt werden.

Das Verfahren aus FIG. 4 kann insbesondere von mindestens einem Prozessor basierend auf Programmcode, der aus mindestens einem Speicher geladen ist, ausgeführt werden.

Das Verfahren aus FIG. 4 kann von einem chirurgischen Assistenzsystem während des chirurgischen Eingriffs ausgeführt werden.

FIG. 4 dient einerseits der Anpassung einer initialen Patienten-Registrierung während eines Beobachtungszeitraums. Die initiale Patienten-Registrierung kann zum Beispiel mittels des Verfahrens aus FIG. 3, dort insbesondere Box 3015, erhalten werden. Das Verfahren aus FIG. 4 kann sich also an das Verfahren aus FIG. 3 anschließen. Die Anpassung der initialen Patienten-Registrierung erfolgt indirekt, basierend auf einem Hilfskoordinatensystem. Das bedeutet also in anderen Worten, dass die Patienten-Registrierung in FIG. 4 geschätzt wird. Das Verfahren aus FIG. 4 dient entsprechend andererseits auch der Bestimmung von Kontextinformation für diese Schätzung der Patienten-Registrierung. Die Kontextinformation kann zum Beispiel alternative Schätzung der Patienten-Registrierung umfassen; und/oder Information zu Unsicherheit der Marker-basierten Schätzung der Patienten-Registrierung. Die Kontextinformation kann basierend auf in den intraoperativen Bilddaten dargestellten anatomischen Landmarken erfolgen. Insbesondere kann eine der in Tab. 1 dargestellt Techniken verwendet werden.

Das Verfahren aus FIG. 4 weist zwei parallele iterative Prozesse 3001, 3002 auf. Der iterative Prozess 3001 entspricht dabei der Marker-basierten Schätzung der Patienten-Registrierung; im Rahmen des iterativen Prozesses 3002 wird die zugehörige Kontextinformation ermitteln.

Während im FIG. 4 die zwei parallelen iterativen Prozesse 3001, 3002 separat dargestellt sind, wäre es grundsätzlich möglich, dass anstatt von zwei parallelen iterativen Prozessen 3001, 3002 verschiedene Boxen aus FIG. 4 in einem gemeinsamen Prozess, zum Beispiel sequenziell, ausgeführt werden.

Im iterativen Prozess 3001 wird basierend auf Referenzmarkern (vgl. FIG. 1: Marker 892), die lagefest an einer Halterung für einen Patienten befestigt sind, die Lage dieser Halterung während eines Beobachtungszeitraums im globalen Referenz-Koordinatensystem des Navigationssystems gemessen bzw. über mehrere Iterationen des iterativen Prozesses 3001 nachverfolgt.

Die Halterung kann beispielsweise direkt am Skelett eines Patienten befestigt sein. Beispielweise kann es sich um eine Kopf-Klemme handeln.

Die Referenzmarker können in den verschiedenen hierin offenbarten Beispielen zum Beispiel aktive oder passive Referenzmarker sein. Es können beispielsweise Leuchtmarker oder Radiomarker oder Infrarot-sensitive Marker verwendet werden. Die Referenzmarker sind künstliche Objekte. Im Navigationssystem kann Vorwissen über die Referenzmarker vorhanden sein, beispielsweise betreffend deren relative Orientierung zueinander und deren Aussehen. Die Referenzmarker können eine sechsdimensionale Bestimmung der Lage der Halterung bzw. des entsprechenden lokalen Halterung-Koordinatensystems 92 (vergleiche FIG. 1) ermöglichen.

Die Bestimmung und Nachverfolgung der Lage der Halterung in Box 3105 dient als Hilfsmittel für die Bestimmung der Lage des lokalen Patienten-Koordinatensystems.

In Box 3110 wird eine aktualisierte Schätzung der Patienten-Registrierung bestimmt. Wird beispielsweise in mehreren Iterationen des iterativen Prozesses 3001 eine geänderte Lage der Halterung in Box 3105 bestimmt, so kann die entsprechend geänderte Lage der Halterung übertragen werden in eine Änderung der Patienten-Registrierung (ausgehend von der initialen Patienten-Registrierung aus Box 3015 oder einem anderen vorgegeben Wert für die Patienten-Registrierung). Dies ist also eine relative Schätzung der Patienten-Registrierung. Deshalb akkumulieren sich bei einem solchen relativen Schätzungsverfahren etwaige systematische Fehler aufgrund eines Drifts der Lage der Halterung gegenüber der Lage der Eingriffsregion des Patienten.

Um einen solchen Fehler zu reduzieren oder zu quantifizieren wird der iterative Prozess 3002 ausgeführt. Dieser wird als nächstes erklärt.

In Box 3150 werden intraoperative Bilddaten von einem Operationsmikroskop erhalten. In jeder Iteration des iterativen Prozesses 3002 können ein oder mehrere Bilder erhalten werden. Beispielsweise wäre es denkbar, dass pro Iteration des iterativen Prozesses 3002 jeweils ein Paar von stereoskopischen Bildern (linker Kanal und rechter Kanal) vom Operationsmikroskop erhalten werden. In einer Variante wäre es denkbar, dass lediglich ein Bild in Box 3150 erhalten wird, beispielsweise zusammen mit Tiefen-Information, die von einem Tiefensensor (beispielsweise einem Ultraschallsensor oder einem stereoskopisch arbeitenden Sensor oder einem Lichtpuls-basierten Laufzeitsensoren oder einem Radarsensor) erhalten wird. Es könnte auch pro Iteration des iterativen Prozesses 3002 eine Zeitabfolge von solchen Bildpaar nur noch einzelnen Bildern erhalten werden.

In Box 3155 erfolgt eine Bildanalyse der zuvor erhaltenen intraoperativen Bilddaten. Dies erfolgt, um ein oder mehrere in den intraoperativen Bilddaten dargestellte anatomische Landmarken, die in Bezug auf das Skelett lagefest sind, aufzufinden.

In Box 3160 wird überprüft, ob eine oder mehrere geeignete anatomische Landmarken in den intraoperativen Bilddaten gefunden wurden. Ist dies nicht der Fall, so kann keine Kontextinformation für die Schätzung der Patienten-Registrierung ermittelt werden und Box 3150 wird in einer weiteren Iteration des iterativen Prozesses 3002 für nachfolgende intraoperativen Bilddaten durchgeführt ("Nein"-Zweig auf Box 3160). Wenn andererseits ein oder mehrere geeignete anatomischen Landmarken aufgefunden wurden, so wird Box 3165 ausgeführt.

In Box 3165 wird die Kontextinformation für die Schätzung der Patienten-Registrierung bestimmt.

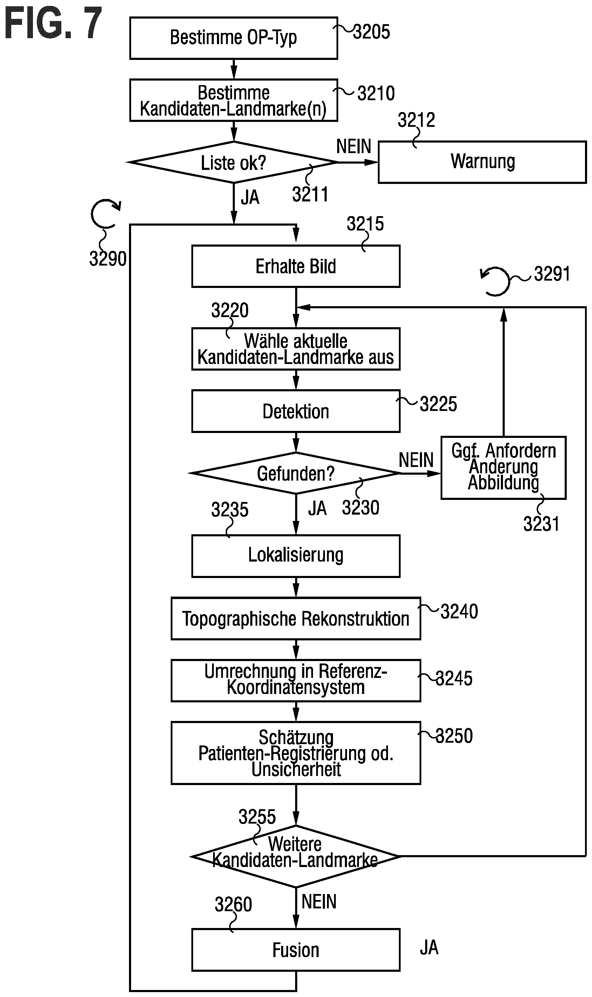

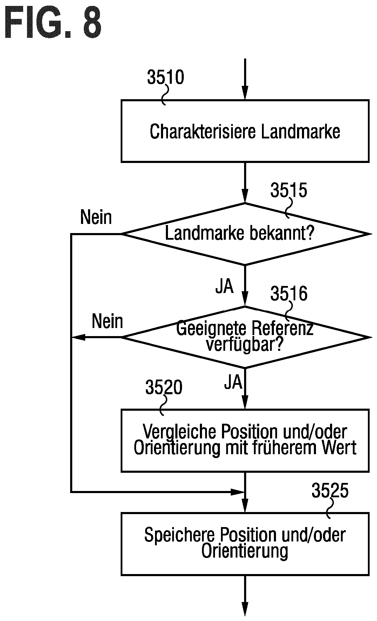

Dies erfolgt anhand einer oder mehrerer der Techniken aus Tab. 1. Mehrere beispielhafte Implementierungen - die beispielsweise die Bestimmung der Position und/oder Lage der in Box 3155 gefundenen Landmarke basierend auf intraoperativen Bilddaten beinhalten kann - wird später (beispielsweise im Zusammenhang mit FIG. 7 und FIG. 8) im Detail studiert.

Insbesondere wird bei der Technik gemäß Tab. 1: Beispiel 1, die aktuell gültige Schätzung der Patienten-Registrierung aus Box 3110 als a-priori Schätzung benötigt. Dies ist durch den entsprechenden gestrichelten Pfeil, der die Box 3110 mit der Box 3165 verbindet, dargestellt. Bei der Technik gemäß Tab. 1: Beispiel 2 wird die aktuelle Schätzung der Patienten-Registrierung aus dem iterativen Prozess 3001 aber nicht zwingend benötigt.

In Box 3170 kann die zuvor bestimmte Kontextinformation verwendet werden. Dazu wird in Box 3170 ein Steuersignal bestimmt. Je nach Informationsgehalt der Kontextinformation sind unterschiedliche Anwendungsszenarien und Ausprägungen des Steuersignals denkbar. So kann beispielsweise mittels des Steuersignals eine Benutzerschnittstelle angesteuert werden - beispielsweise eine graphische Benutzerschnittstelle oder eine Audio-Benutzerschnittstelle -, um eine Beurteilung der Schätzung der Patienten-Registrierung an einen Benutzer auszugeben. Ein Soll-Ist-Vergleich kann visualisiert werden. Es könnte z.B. die Abweichung, z.B. in Längeneinheiten wie Millimeter ausgegeben werden. Beispielsweise könnte bei einer Beurteilung, die eine hohe Ungenauigkeit oder Unsicherheit der Schätzung der Patienten-Registrierung ergibt, eine Warnung an den Benutzer über eine Benutzerschnittstelle ausgegeben werden. Es wäre aber auch denkbar, dass bei Verfügbarkeit einer alternativen Schätzung der Patienten-Registrierung aus Box 3165 anhand der intraoperativen Bilddaten das entsprechende Steuersignal an das Navigationssystem gesendet wird, welches den iterativen Prozess 3001 ausführt (gestrichelte Pfeil, der Box 3170 mit Box 3110 verbindet; wobei dort ein entsprechendes Steuersignal 971, welches Kontextinformation 970 indiziert, schematisch gezeigt ist). Das Navigationssystem könnte dann die aktualisierte Schätzung in Box 3110 gemäß dem Steuersignal verwenden oder einen Mittelwert bilden oder im Rahmen eines Kalman-Filters berücksichtigen, um nur einige Beispiele zu nennen.

In den verschiedenen Beispielen kann die Kontextinformation Ortsinformation beinhalteten. Dies bedeutet, dass die Kontextinformation ortsaufgelöst bestimmt wird. Entsprechend kann das Steuersignal ein oder mehrere Aktionen anfordern, die eine Steuerung unter Berücksichtig des Orts bewirken.

Wie voranstehend erläutert, wird die Kontextinformation anhand von ein oder mehreren anatomischen Landmarken bestimmt, die in den intraoperativen Bilddaten abgebildet werden. Die entsprechende Kontextinformation weist also eine Gültigkeit insbesondere bei und in der Nähe der jeweiligen anatomischen Landmarke auf. Beispielsweise könnte die Kontextinformation eine graduelle Gültigkeit aufweisen, die umso größer bzw. kleiner ist, je näher bzw. weiter entfernt die entsprechende Position von der entsprechenden anatomischen Landmarke auf deren Grundlage die Kontextinformation bestimmt wird ist.

So kann es insbesondere bei relativ großen Gesichtsfeldern des Operationsmikroskops bzw. der intraoperativen Bilddaten vorkommen, dass die entsprechende Kontextinformation in anderen Bereichen der intraoperativen Bilddaten, das heißt entfernt von der jeweiligen anatomischen Landmarke, keine oder nur eine eingeschränkte Gültigkeit aufweist. Dies kann durch das ortsaufgelöste Bestimmen der Kontextinformation berücksichtigt werden.

Es wäre alternativ oder zusätzlich auch denkbar, dass die Konfidenz einer Bestimmung der Position und/oder Orientierung der anatomischen Landmarke in den intraoperativen Bilddaten bei der Bestimmung der Gültigkeit bzw. Konfidenz der Kontextinformation berücksichtigt wird. Basierend auf einer solchen Konfidenz der Position und/oder Orientierung der anatomischen Landmarke kann dann noch eine Konfidenz für die Kontextinformation bestimmt werden.

Beispielsweise wäre es denkbar, dass bei einer ortsaufgelöst bestimmten Kontextinformation die Benutzerschnittstelle mittels des entsprechenden Steuersignals angesteuert wird, um lokale Bereiche innerhalb des Gesichtsfelds in denen die Kontextinformation eine hohe Konfidenz bzw. Gültigkeit aufweist, anzuzeigen.

Obenstehend wurden Varianten beschrieben, bei denen die Kontextinformation mit einer bestimmten Gültigkeit bzw. Konfidenz versehen ist. Es ist entsprechend denkbar, dass die Kontextinformation selbst die Konfidenz der Patienten-Registrierung ortsaufgelöst anzeigt. Beispielsweise kann mittels der Kontextinformation bestimmt werden, dass die Patienten-Registrierung eine geringe Konfidenz aufweist. Dies kann entsprechend kombiniert werden mit einer lokal gültigen Kontextinformation, so dass die Patienten-Registrierung in einem bestimmten Bereich eine geringe Konfidenz aufweist.

Beispielsweise wäre es denkbar, dass bei einer ortsaufgelöst bestimmten Kontextinformation die Benutzerschnittstelle mittels eines entsprechenden Steuersignals angesteuert wird, um lokale Bereiche innerhalb des Gesichtsfelds der intraoperativen Bilddaten zu indizieren, die mit einer besonders hohen oder niedrigen Konfidenz für die Patienten-Registrierung assoziiert sind. Alternativ oder zusätzlich wäre es möglich, dass eine Anzeige einer Warnung lokal dort erfolgt, wo erwartet wird, dass die verschlechterte Genauigkeit der Navigation negative Auswirkungen auf die Durchführung des operativen Eingriffs haben kann. Beispielsweise könnte die Warnung selektiv an einem Ort erfolgen, an dem Gewebe entfernt werden soll. Die Warnung kann also mit einem Szenen-Detektionsmodell, welches semantischen Kontext für die beobachtete Szene bereitstellt, durchgeführt werden. Solche Techniken beruhen auf der Erkenntnis, dass eine Sparsamkeit bei der Ausgabe von Warnung und die Priorisierung von Einblendungen die kognitive Last auf den Chirurgen senken kann.

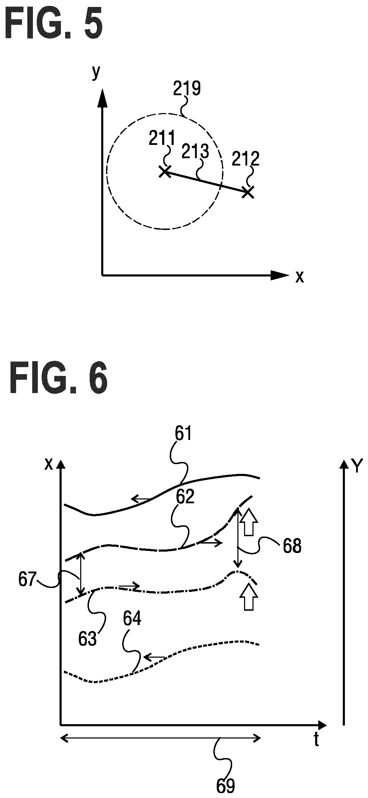

In Box 3165 können, wie voranstehend beschrieben, Techniken angewendet werden, die in Tab. 1 aufgelistet sind. Als nächstes werden weitere Details diesen Techniken im Zusammenhang mit FIG. 5 (Tab. 1: Beispiel 1) sowie FIG. 6 (Tab. 1: Beispiel 2) erläutert.

FIG. 5 illustriert eine beispielhafte Implementierung von TAB. 1: Beispiel 1. Die Bestimmung der aktuellen Position 211 (in FIG. 5 ist beispielhaft und zum Zwecke der besseren Darstellbarkeit die 2-D Position gezeigt; entsprechende Techniken können aber auch für die 3-D Position und/oder die Orientierung der anatomischen Landmarke angewendet werden) der anatomischen Landmarke im Referenz-Koordinatensystem 91 wird zunächst die anatomische Landmarke in den intraoperativen Bilddaten lokalisiert; dazu werden die intraoperativen Bilddaten beispielsweise während der Operation von einem Operationsmikroskop wie dem Operationsmikroskop 802 erhalten. Beispielsweise könnte die anatomische Landmarke in intraoperativen Bilddaten mittels einer Bounding-Box lokalisiert werden. Es wäre aber auch denkbar, dass eine Segmentierung erfolgt. Es könnte eine Mittelpunktbestimmung oder eine Schwerpunktbestimmung erfolgen.

Über die Kenntnis der Lage der von den intraoperativen Bilddaten abgebildeten Objektebene im globalen Referenz-Koordinatensystem kann dann diese Position der Landmarke in das globale Referenz-Koordinatensystem übertragen werden. Das ist die Position 211 in FIG. 5.

Anhand der derzeit gültigen Patienten-Registrierung 85 (vergleiche FIG. 4: Pfeil von Box 3110 zu Box 3165) kann eine Überlagerung mit entsprechender, aus dem präoperativen Volumenbilddaten abgeleiteter Lokalisierungsinformation für dieselbe anatomische Landmarke erfolgen. Die entsprechende Position 212 ist in FIG. 5 dargestellt. Die Abweichung 213 zwischen der beobachteten Position 211 und der Referenz-Position 212 ist indikativ für eine Unsicherheit oder einen Fehler der Schätzung der Patienten-Registrierung sein. Die Kontextinformation kann das anzeigen.

Beispielsweise wäre es denkbar, dass die Abweichung 213 mit einem Schwellenwert 219 verglichen wird. Im Beispiel der FIG. 5 ist die Abweichung 213 größer als der Schwellenwert. Dies könnte bedeuten, dass anhand der Kontextinformation eine Warnung an einen Benutzer aufgrund der erhöhten Unsicherheit der Patienten-Registrierung ausgegeben wird. Eine solche Warnung könnte zum Beispiel durch eine Überlagerung einer entsprechenden Fehlermeldung in das auf einem Bildschirm angezeigte intraoperative Bild erfolgen. Alternativ oder zusätzlich könnte ein akustisches Warnsignal ausgegeben werden.

Ein solcher beim Vergleich der Schätzung der Position (und/oder Orientierung) der anatomischen Landmarke mit einer vorgegebenen Referenz-Position (und/oder Referenz-Orientierung) berücksichtigt Schwellenwert kann in Abhängigkeit von ein oder mehreren statischen oder dynamischen Kriterien eingestellt werden. Beispielsweise könnte der Schwellenwert einmal vor Beginn eines operativen Eingriffs in Abhängigkeit von Typinformation, die den Typ des operativen Eingriffs angibt, eingestellt werden. Es wäre auch denkbar, dass je nach Fortschritt des operativen Eingriffs unterschiedliche Schwellenwerte verwendet werden. Das bedeutet also, dass eine dynamische Einstellung des Schwellenwerts während des operativen Eingriffs erfolgen kann. Beispielsweise könnten unterschiedliche Schwellenwerte je nach Vorhersage für einen gegenwärtigen oder nachfolgenden Operationsschritt des operativen Eingriffs verwendet werden. Eine Vorhersage für den Operationsschritt kann zum Beispiel basierend auf einem semantischen Verständnis für die dargestellte Szene erfolgen. Beispielsweise könnte eine Kombination der erkannten Landmarken mit dem gespeicherten Operationstyp zusammen mit einem vordefinierten grundsätzlichen Ablaufschema des operativen Eingriffs und gegebenenfalls eine Erkennung von bestimmten Operationsinstrumenten für die Vorhersage genutzt werden. Solche Techniken beruhen auf der Erkenntnis, dass bei bestimmten Phasen eines operativen Eingriffs eine höhere Konfidenz in die Navigation erforderlich ist, als bei anderen Phasen. Manche Phasen eines operativen Eingriffs sind besonders kritisch, beispielsweise weil die Gefahr einer Beschädigung von umliegendem Gewebe besteht. In solchen Szenarien kann es hilfreich sein, bei diesem Vergleich striktere Kriterien zu fordern.

In Zusammenhang mit FIG. 5 sind obenstehend Aspekte beschrieben, bei denen eine zweidimensionale Positionierung (X-Y-Position in FIG. 5) der anatomischen Landmarke erfolgt. Allgemein wäre es möglich, dass die Positionierung nicht nur 2-D erfolgt, sondern höherdimensional. Beispielsweise kann eine 6-D Lage der anatomischen Landmarke anhand intraoperativen Bilddaten geschätzt werden. Eine 6-D Lage bedeutet insbesondere die 3-D Position ("xyz-Position") sowie eine 3-D Orientierung (z.B. "Nicken-Gieren-Rollen").

Als nächstes wird ein konkretes Beispiel für das Szenario aus FIG. 5 erläutert. Als anatomische Landmarke wird der optische Nerv bei einer kranialen Operation verwendet. Der optische Nerv wird im linken 2-D Bild des stereoskopischen Operationsmikroskops erkannt. Anschließend wird Lokalisierungsinformation für den optischen Nerv bestimmt. Das linke 2-D Bild wird dazu segmentiert. Dazu könnte zum Beispiel ein speziell trainiertes maschinengelerntes Modell verwendet werden: ein solches speziell trainiertes maschinengelerntes Modell kann eingerichtet sein, um ausschließlich den optischen Nerv in Bilddaten, die von einem Operationsmikroskop erhalten werden, zu segmentieren. In einer alternativen Variante wäre es aber auch denkbar, dass ein generisches maschinengelerntes Modell verwendet wird. Dabei können unterschiedliche Stufen der Generalisierung beim Training des maschinengelernten Modells berücksichtigt werden. In einer ersten Stufe könnte das maschinengelernte Modell trainiert sein, um unterschiedliche Typen von anatomischen Landmarken in Bildern, die von einem Operationsmikroskop erhalten werden, zu segmentieren. In einer zweitens Stufe der Generalisierung könnte das maschinengelernte Modell unspezifisch für Bilder von einem Operationsmikroskop sein. Beispielsweise könnte ein sogenanntes vom Foundation-Modell verwendet werden, welches eine Segmentierung von unterschiedlichsten Typen von Objekten in unterschiedlichsten Typen von Bildern durchführen kann. In einem solchen Szenario könnte ein Text-Prompt an das Domänen-unspezifische Segmentierungsmodell übergeben werden, welcher das Modell beauftragt, den optischen Nerv zu segmentierten.

Eine Segmentierungsmaske wird bestimmt, welche den optischen Nerv von anderen Strukturen bzw. Hintergrund trennt. Mittels Stereoskopie, basierend auf einem Vergleich des linken 2-D Bilds des stereoskopischen Operationsmikroskops mit dem rechten 2-D Bild des stereoskopischen Operationsmikroskops, erfolgt eine topographische Rekonstruktion der 3-D Oberfläche des optischen Nervs, das heißt zu jedem Pixel existiert im Anschluss ein Tiefenwert. Alternativ wird die 3-D Oberfläche des optischen Nervs als Gitter repräsentiert; es kann eine 6-D Lagebestimmung des optischen Nervs erfolgen. Dabei kann auch die intrinsische und extrinsische Kalibration des Operationsmikroskops verwendet werden, so dass die 3-D Oberfläche in einem absoluten Referenz-Koordinatensystem (beispielsweise in Einheiten von Millimetern) vorliegt, und nicht nur in der Bildebene (dort dann ausgedrückt in Pixeln). Insbesondere könnte die 3-D Oberfläche des optischen Nervs in einem Referenz-Koordinatensystem des Operationsmikroskops vorliegen, dass mit dem globalen Referenz-Koordinatensystem des Navigationssystems registriert ist. Entsprechend kann das Navigationssystem angefragt werden, die 3-D Oberfläche des optischen Nervs im Referenz-Koordinatensystem des Operationsmikroskops an das Navigationssystem zu übermitteln, um diese 3-D Oberfläche im globalen Referenz-Koordinatensystem des Navigationssystems auszudrücken (vgl. globale Referenz-Koordinatensystem 91 in FIG. 2). Es wird angenommen, dass die präoperativen Volumenbilddaten bezüglich der verschiedenen anatomischen Landmarken und insbesondere des optischen Nervs segmentiert sind und diese Segmentierungsmasken - basierend auf der derzeitigen Schätzung der Patienten-Registrierung - im globalen Referenz-Koordinatensystem angeordnet sind. Sind mehrere anatomischen Landmarken desselben Typs vorhanden, kann können diese aufgrund ihrer unterschiedlichen Position und Orientierung im globalen Referenz-Koordinatensystem voneinander unterschieden werden. Mittels eines Registrierungsverfahrens, zum Beispiel iterative closests points, können die 3-D Oberflächen des optischen Nervs, die einmal aus den intraoperativen Bilddaten bestimmt sind und einmal aus den präoperativen Bilddaten bestimmt sind, miteinander registriert werden. Die entsprechende Registrierung ist indikativ für den Abstand 213 der 6-D Lagen. Daraus resultiert eine Transformationsmatrix, welche Translationskomponenten und Rotationskomponente beinhaltet. Daraus kann die derzeitige Genauigkeit der Navigation, das heißt die Unsicherheit der vom Navigationssystem verwendeten Schätzung der Patienten-Registrierung, bestimmt werden.

Im Beispiel aus FIG. 5 bzw. TAB. 1: Beispiel 1 ist es notwendig, die anatomische Landmarke auch in den präoperativen Volumenbilddaten zu lokalisieren. Dies ist erforderlich, um die Referenz-Position 212 zu bestimmen. Manchmal kann es allerdings vorkommen, dass keine präoperativen Volumenbilddaten verfügbar sind. Es könnte auch vorkommen, dass bestimmte anatomische Landmarken in den präoperativen Bilddaten nicht oder nur eingeschränkt sichtbar sind bzw. nicht gut lokalisiert werden können. In solchen Fällen ist insbesondere eine Technik gemäß FIG. 6 bzw. TAB. 1: Beispiel 2 hilfreich. Diese werden nachfolgend erläutert.

FIG. 6 illustriert Aspekte in Bezug auf TAB. 1: Beispiel 2. FIG. 6 illustriert eine zeitliche Entwicklung der X-Position und der Y-Position für das Marker-Koordinatensystem (X-Position: Kurve 61; Y-Position: Kurve 62) sowie für das anhand der intraoperativen Bilddaten geschätzte lokale Patienten-Koordinatensystem (X-Position: Kurve 64; Y-Position Kurve 63). Die Kurven 63 und 64 werden anhand der Position einer in den intraoperativen Bilddaten positionierten und nachverfolgten Landmarke bestimmt.

Im in FIG. 6 dargestellten Beispiel ist die zeitliche Abhängigkeit der X-Position (Kurve 61 und Kurve 64) für das lokale Marker-Koordinatensystem und die anatomische Landmarke gleich. Allerdings kann für die zeitliche Abhängigkeit der Y-Position (Kurve 62 und Kurve 63) eine Abweichung zwischen dem lokalen Marker-Koordinatensystem und der anatomischen Landmarke beobachtet werden (in FIG. 6, mit den Pfeilen markiert).

Allgemein kann ein Vergleich zwischen zwei Größen durchgeführt werden, sodass die Änderung der Marker-basierten Schätzung der Patienten-Registrierung 83 während des Beobachtungszeitraums 69 in Bezug gesetzt wird zu der Änderung der Position und/oder Orientierung der anatomischen Landmarke. Im Beispiel der FIG. 6 könnte dies bedeuten, dass die Differenz 67 zu Beginn des Beobachtungszeitraums 69 verglichen wird mit der Differenz 68 zum Ende des Beobachtungszeitraums 69. Da diese Differenzen 67, 68 voneinander abweichen, bedeutet dies, dass sich die aus der Schätzung der Patienten-Registrierung 83 (diese basiert auf der Kurve 64) abgeleitete Y-Position während des Beobachtungszeitraums 69 anders verändert als die tatsächlich beobachtete Y-Position (Kurve 62) der anatomischen Landmarke. Anstatt eines Vergleichs der Position und/oder Orientierung aus der Schätzung der Patienten-Registrierung zu einem ersten Zeitpunkt mit der Position und/oder Orientierung aus den intraoperativen Bilddaten zu einem zweiten Zeitpunkt durchzuführen (wie in FIG. 6 für die Y-Position anhand der Differenz 67 und der Differenz 68 gezeigt), wäre es auch möglich, zum Beispiel die zeitlichen Änderungsraten beider Kurven kontinuierlich in Bezug zu setzen. Es sind allgemein viele äquivalente Implementierungsvarianten für einen solchen Vergleich, der die Änderung der Schätzung der Patienten-Registrierung während des Beobachtungszeitraums in Bezug setzt zu der Änderung der Position und/oder Orientierung der anatomischen Landmarke während des Beobachtungszeitraums, denkbar.

Auch bei der Variante aus FIG. 6 kann der Vergleich einen Schwellenwert berücksichtigen. Hier gelten entsprechende Ausführungen, wie sie im Zusammenhang mit dem Schwellenwert 219 im Zusammenhang mit FIG. 5 gemacht wurden. Beispielsweise könnte im in FIG. 6 dargestellten Szenario überprüft werden, ob der Betrag der Differenz zwischen (i) der Differenz 67 und (ii) der Differenz 68 größer oder kleiner als der Schwellenwert ist. Bestimmte kleine Unterschiede der Änderung können derart toleriert werden; während größere Unterschiede der Änderung zu einer Korrektur der Warnung führen können.

Sowohl die in FIG. 5 als auch die in FIG. 6 beschriebenen Techniken zur Bestimmung der Kontextinformation zur Schätzung der Patienten-Registrierung beruhen auf der Bestimmung der Positionierung ein oder mehrerer anatomischer Landmarken in und anhand der intraoperativen Bilddaten. Verschiedenen hierin beschriebenen Techniken beruhen auf der Erkenntnis, dass es zur Bestimmung von aussagekräftiger Kontextinformation hilfreich ist, geeignete anatomischer Landmarken in den intraoperativen Bilddaten zu lokalisieren. Nicht jeder anatomische Landmarke ist besonders geeignet, um aussagekräftige Kontextinformation zu bestimmen. Verschiedene Techniken beruhen auf der Erkenntnis, dass manche anatomische Landmarken oftmals nur teilweise in den intraoperativen Bilddaten sichtbar sind. Manche anatomischen Landmarken sind nur vergleichsweise kurz in den intraoperativen Bilddaten sichtbar, und für den Rest der Zeit des chirurgischen Eingriffs verdeckt. Ferner wurde beobachtet, dass es anhand bestimmter anatomischer Landmarken nicht oder nur eingeschränkt möglich, basierend auf der 2-D Abbildung der anatomischen Landmarke in den intraoperativen Bilddaten auf eine 6-D Lage der anatomischen Landmarke, das heißt deren Position und Orientierung, zurückzuschließen. In anderen Worten kann für bestimmte anatomische Landmarke eine topographische Rekonstruktion oder Posen-Rekonstruktion fehlschlagen oder nur mit einer relativ hohen Ungenauigkeit assoziiert sein. In FIG. 7 sind entsprechend Techniken dargestellt, welche eine robuste Bestimmung von aussagekräftiger Kontextinformation anhand der geeigneten Auswahl von ein oder mehreren anatomischen Landmarken ermöglichen.

FIG. 7 ist ein Flussdiagramm eines beispielhaften Verfahrens. Das Verfahren aus FIG. 7 betrifft die Auswertung von intraoperativen Bilddaten. Das Verfahren aus FIG. 7 ist eine beispielhafte Implementierung von Box 3155, Box 3160 sowie Box 3165 in FIG. 4.

In Box 3205 wird ein Typ des operativen Eingriffs bestimmen. Beispielsweise kann entsprechende Typinformation ermittelt werden. Die Typinformation kann zum Beispiel zweistufig bestimmt werden. Beispielsweise könnte eine Vorauswahl des Operationstyps auf einer hohen Abstraktionsebene erfolgen und dann die konkrete Zugangsart in einer zweiten Stufe ausgewählt werden. Die Typinformation kann beispielsweise mehrere Hierarchieebenen aufweisen. Eine erste Hierarchieebene kann zum Beispiel die Eingriffsregion spezifizieren, also zum Beispiel angeben: Kopfbereich; Wirbelsäule; Knie; Zahn, Ohr, Hals, Hand, etc. Die zweite Hierarchieebene könnte zum Beispiel eine Orientierung des Zugangskanal innerhalb der jeweiligen Eingriffsregion für den jeweiligen Operationstool spezifizieren. Beispielsweise sind im Operationsbereich unterschiedlichen Orientierungen des Zugangskanals denkbar, je nachdem von welcher Seite und unter welcher Orientierung der Zugang gelegt wird.

Anhand solcher Typinformation bzw. allgemein anhand von Information zum Typ des operativen Eingriffs kann dann in Box 3210 eine Liste mit ein oder mehreren anatomischen Kandidaten-Landmarken erzeugt werden. Diese sind Kandidaten für die Erzeugung der Kontextinformation. Eine solche Liste kann erlaubte Kandidaten-Landmarken ("white listing") und/oder verbotene Kandidaten-Landmarken ("black listing") umfassen. Solche Techniken beruhen auf der Erkenntnis, dass es je nach Typ des operativen Eingriffs unterschiedliche Landmarken gibt, die grundsätzlich für die Bestimmung der Kontextinformation infrage kommen. Beispielsweise können bestimmte Typen von anatomischen Landmarken nur für bestimmte Typen von operativen Eingriffen beobachtet werden. Wenn zum Beispiel ein frontaler Zugangskanal verwendet wird, können keine anatomischen Landmarken beobachtet werden, die bei einem Zugang durch den Nacken beobachtbar wären. Ferner können bestimmte anatomischen Landmarken gut für eine entsprechende Bildauswertung geeignet ein, beispielsweise weil sich eine 6-D Lage der Landmarke besonders robust und zuverlässig anhand der intraoperativen Bilddaten für eine solche anatomischen Landmarke rekonstruieren lässt. Entsprechend kann es vorkommen, dass andere anatomischen Landmarken nicht besonders gut für eine entsprechende Posen-Rekonstruktion oder Rekonstruktion der Topographie geeignet sind, zum Beispiel weil sie typischerweise mit einem geringen Kontrast in den intraoperativen Bilddaten erscheinen. Manche anatomischen Landmarken können auch nur mit einer geringen Wahrscheinlichkeit in den intraoperativen Bilddaten sichtbar sein, zum Beispiel weil sie bei einem bestimmten Operationstyp in der Regel über einen ausgedehnten Zeitraum verdeckt oder zumindest teilweise verdeckt sind. Solcher anatomischen Landmarken sind dann weniger geeignet. Solche und weitere Faktoren können bei der Bestimmung einer Liste mit ein oder mehreren Kandidaten-Landmarken basierend auf Typinformation für den operativen Eingriff berücksichtigt werden.

Es wäre denkbar, dass eine entsprechende Liste mit Kandidaten-Landmarken auch Priorisierungsinformation für die beinhalteten Kandidaten-Landmarken umfasst. Eine solche Priorisierungsinformation kann eine relative Priorität der verschiedenen Kandidaten-Landmarken in der Liste zueinander angeben. So könnten solche Kandidaten-Landmarken mit einer höheren Priorität versehen sein, die mit einer höheren Wahrscheinlichkeit über einen ausgedehnten Zeitraum sichtbar sind. Entsprechend wäre es denkbar, dass solche Kandidaten-Landmarken mit einer höheren Priorisierungsinformation assoziiert sind, die sich besonders gut lokalisieren lassen oder die besonders geeignet für eine Rekonstruktion der 6-D Lage sind. Auch die Priorisierungsinformation kann, gemäß den oben dargelegten Abhängigkeiten, aus der Typinformation bestimmt werden.

Für die verschiedenen Kandidaten-Landmarken, die sich in der Liste befinden, kann Vorwissen zu deren Erscheinungsbild in den intraoperativen Bilddaten vorhanden sein. Anhand von solchem Vorwissen kann eine zuverlässige Detektion, Lokalisierung und/oder Rekonstruktion von Lageparametern erfolgen. Insbesondere im Vergleich zu Referenztechniken, bei denen eine anatomische Landmarke opportunistisch bei Erscheinen in den intraoperativen Bilddaten ausgewählt wird, kann derart eine gewisse Robustheit im Zusammenhang mit der Bestimmung der Kandidaten Information sichergestellt werden. Außerdem wird kein manueller Eingriff durch einen Chirurgen zur Bestimmung der entsprechenden anatomischen Landmarke notwendig.

In Box 3211 kann zunächst optional überprüft werden, ob die Liste aus Box 3210 ein oder mehrere vorgegebene Kriterien erfüllt. Beispielsweise könnte überprüft werden, ob die Anzahl der in der Liste vorhandenen erlaubten Kandidaten-Landmarken einen bestimmten Schwellenwert überschreitet. Wenn die Anzahl der Kandidaten-Landmarken den Schwellenwert nicht überschreitet, so könnte in Box 3212 eine Warnung ausgegeben werden. Solche Techniken beruhen auf der Erkenntnis, dass es in verschiedenen Beispielen erforderlich sein kann, eine bestimmte Mindestanzahl von Kandidaten-Landmarken in der Liste aufzunehmen, um derart eine robuste Bestimmung der Kontextinformation und des Steuersignals zu ermöglichen. Der entsprechende Schwellenwert könnte zum Beispiel vom Operationstyp und/oder einer gewünschten Konfidenz für die Navigation abhängen.

Wenn die Liste mit den anatomischen Kandidaten-Landmarken ein oder mehrere entsprechende vorgegebene Kriterien erfüllt, so kann anschließend Box 3215 durchgeführt werden. Der operative Eingriff beginnt.

In Box 3215 wird ein Bild der intraoperativen Bilddaten erhalten. Es könnten aber auch mehrere Bilder erhalten werden, entweder für unterschiedliche stereoskopische Perspektive und/oder einer Zeitabfolge.

In Box 3220 wird eine zu detektierende Kandidaten-Landmarke aus der Liste von Kandidaten-Landmarken ausgewählt (aus Box 3210).

In Box 3225 wird dann ein Algorithmus zur Detektion der jeweiligen aktuellen anatomischen Landmarke in dem aktuellen Bild aus der gegenwärtigen Iteration 3290 von Box 3215 ausgeführt. Beispielsweise könnte ein maschinengelerntes Modell ausgeführt werden, welches eine Wahrscheinlichkeit für die Anwesenheit einer entsprechenden anatomischen Landmarke in dem jeweiligen aktuellen Bild ausgibt. Das Detektionsmodell muss dabei keine Lokalisierungsinformation für die aktuelle Kandidaten-Landmarke ausgeben.

Das maschinengelernte Modell kann also eine automatisierte, bildbasierte Erkennung von anatomischen Landmarken ermöglichen. Das maschinengelernte Modell kann in verschiedenen operativen Eingriffen verwendet werden. Das bedeutet, dass ein einmaliges Training durchgeführt werden kann, welches Gültigkeit für verschiedene operative Eingriffe behält. Insbesondere kann im Rahmen einer Detektionsaufgabe ein relativ robustes Anwenden desselben maschinengelernten Modells auf Bilddaten aus unterschiedlichen operativen Eingriffen, gegebenenfalls aus unterschiedlichen Bildquellen, ermöglicht werden. Eine Patienten-spezifische Anpassung des maschinengelernten Modells muss nicht erfolgen.

Es kann also eine kontinuierliche Auswertung des Videostroms, das heißt der Zeitsequenz von Bildern der intraoperativen Bilddaten, erfolgen, um jeweils die Präsenz von Landmarken eines bestimmten Typs (gemäß der Liste aus Box 3210) in den Bildern zu bestimmen. Es wäre optional möglich, dass eine Detektion in Box 3225 für einen bestimmten Typ von Kandidaten-Landmarken manuell vom Chirurgen ausgelöst wird, wenn der Chirurg befindet, dass eine entsprechende Landmarke diese Typs sichtbar ist.

In Box 3230 kann überprüft werden, ob das Detektionsmodell die jeweilige Kandidaten-Landmarke (zum Beispiel mit einer hinreichenden Zuverlässigkeit) aufgefunden hat. Ist dies der Fall, wird Box 3235 ausgeführt; andernfalls wird optional Box 3231 und eine weitere Iteration 3291 von Box 3220, für die nächste Kandidaten-Landmarke in der Liste durchgeführt. In der optionalen Box 3231 könnte gegebenenfalls eine Änderung der Konfiguration des Operationsmikroskops, die die Erfassung der intraoperativen Bilddaten reguliert, angefordert werden. Insbesondere könnte eine Vergrößerung des Gesichtsfelds des Operationsmikroskops angefordert werden. Dies kann zum Beispiel durch eine Herabsetzung des Vergrößerungsfaktors erfolgen. Beispielsweise könnte Box 3231 überprüft werden, ob eine bestimmte Kandidaten-Landmarke für einen gewissen Zeitraum nicht sichtbar gewesen ist. Beispielsweise könnte überprüft werden, ob eine bestimmte Kandidaten-Landmarke für eine bestimmte Anzahl von Iterationen 3290 nicht sichtbar gewesen ist. In einem solchen Fall könnte die Erweiterung des Gesichtsfelds des Operationsmikroskops angefordert werden.

Eine solche Anforderung zur Erweiterung des Gesichtsfelds des Operationsmikroskops könnte auch nur dann angefordert werden, wenn eine bestimmte Anzahl von Kandidaten-Landmarken gemeinsam für eine bestimmte Zeitdauer nicht in den intraoperativen Bilddaten detektiert werden kann.

Wenn Box 3235 ausgeführt wird, das heißt es wurde eine bestimmte Landmarke in dem aktuellen Bild aus der gegenwärtigen Iteration 3290 von Box 3215 gefunden, so kann anschließend Lokalisierungsinformation für diese Landmarke bestimmt werden. Die Lokalisierungsinformation in Box 3235 lokalisiert die jeweilige detektierte Landmarke innerhalb des Bilds aus Box 3215. Dies bedeutet also, dass eine 2-D Lokalisierung durchgeführt wird. Die Lokalisierungsinformation kann zum Beispiel eine Bounding-Box oder eine Segmentierungsmaske umfassen.

Beispielsweise kann ein maschinengelerntes Modell verwendet werden, welches speziell für die Bestimmung von Lokalisierungsinformation für den jeweiligen Typ der anatomischen Landmarke trainiert wurde. Die Lokalisierung in Box 3235 kann, allgemein formuliert, basierend auf Vorwissen über das Erscheinungsbild der jeweiligen anatomischen Landmarke erfolgen. Die infrage kommenden anatomischen Landmarken sind vordefiniert (vergleiche Box 3210), so dass entsprechendes Vorwissen vorgehalten werden kann. Es können speziell adaptierte Modelle für die Lokalisierung verwendet werden. Es könnte aber auch ein generisches Segmentierungsmodell verwendet werden, dass nicht Domänen-spezifisch für diesen Typ der anatomischen Landmarke oder gar Bilddaten eines Operationsmikroskops ist, verwendet werden.

In Box 3235 kann ferner ein Tracking für eine Vielzahl von anatomischen Landmarken erfolgen. Beispielsweise wäre es denkbar, dass bestimmte, ähnlich aussehende anatomischen Landmarken, gegebenenfalls desselben Typs, gleichzeitig in den intraoperativen Bilddaten abgebildet sind. Um eine zuverlässige Lokalisierung jeder dieser mehreren anatomischen Landmarken über mehrere Iterationen 3290 hinweg zu ermöglichen, kann es sinnvoll sein, einen Tracking-Algorithmus auszuführen. Dieser erlaubt es, zwischen unterschiedlichen anatomischen Landmarken über mehrere sequenzielle Bilder der intraoperativen Bilddaten hinweg zu differenzieren.

In Box 3240 kann optional eine topographische Rekonstruktion einer Oberfläche der zuvor lokalisierten Landmarke erfolgen. In anderen Worten kann eine 3-D Form und Lage der anatomischen Landmarke basierend auf den niederdimensionalen intraoperativen Bilddaten bestimmt werden. Beispielsweise kann ein sogenanntes Posen-Schätzungsmodell angewendet werden. Basierend auf einer solchen topographischen Rekonstruktion in Box 3240 kann Lageinformation, welche eine Position und Orientierung der 3-D anatomischen Landmarke angibt, bestimmt werden.

Es ist nicht in allen Varianten erforderlich, dass eine topographische Rekonstruktion der Oberfläche der Landmarke stattfindet; das heißt Box 3240 ist optional. In manchen Beispielen wäre es auch denkbar, dass lediglich die Position oder die Orientierung der Landmarke in der Bildebene der intraoperativen Bilddaten verwendet wird. Auch basierend auf solcher niedrigdimensionalen Lokalisierungsinformation aus Box 3235 ist es möglich, Kontextinformation für die Schätzung der Patienten-Registrierung zu bestimmen.

In Box 3245 erfolgt optional eine Umrechnung der Position und/oder Orientierung der Landmarke (basierend auf der Lokalisierungsinformation aus Box 3235 oder basierend auf der Lageinformation aus Box 3240) in ein Referenz-Koordinatensystem; beispielsweise das vom Navigationssystem definierte globale Referenz-Koordinatensystem 91. Die Lage der Objektebene im globalen Referenz-Koordinatensystem ist über die Nachverfolgung der Lage des Operationsmikroskops sowie ein geeignetes Kameramodell des Operationsmikroskops bekannt.

Die Umrechnung in das globale Referenz-Koordinatensystem ist für das Szenario aus Tab. 1: Beispiel 1 hilfreich. Für das Szenario aus Tab. 1: Beispiel 2 ist die Umrechnung in das globale Referenz-Koordinatensystem nicht zwingend erforderlich (obschon möglich). Allerdings sollte sichergestellt werden, dass die Änderung der Position und/oder Orientierung der anatomischen Landmarke durch Vergleich zweier im selben Referenz-Koordinatensystem bestimmten Werte erfolgt; wird beispielsweise des Operationsmikroskops zwischen dem Erfassen zweier Bilder der intraoperativen Bilddaten bewegt, so kann eine entsprechende Kompensation für die Bewegung des Operationsmikroskops erfolgen. Beispielsweise könnte die Position und/oder Lage der Landmarke in ein Referenz-Koordinatensystem des Operationsmikroskops umgerechnet werden.

Anschließend kann dann in Box 3250 basierend auf dem Wissen über die Position und/oder Orientierung der anatomischen Landmarke eine alternative Schätzung für die Patienten-Registrierung bestimmt werden oder zumindest die Unsicherheit der mittels des Marker-Koordinatensystems bestimmten Schätzung der Patienten-Registrierung bestimmt werden, vergleiche Tab. 1, FIG. 5 und FIG. 6.

Wenn die alternative Schätzung für die Patienten-Registrierung bestimmt wird, so kann auch eine Unsicherheit für diese alternative Schätzung der Patienten-Registrierung ermittelt werden. Beispielsweise könnte die Unsicherheit der alternativen Schätzung der Patienten-Registrierung anhand einer Unsicherheit im Zusammenhang mit der Lokalisierung(en) in Box 3235 bestimmt werden. Beispielsweise wäre es denkbar, dass bestimmte anatomische Landmarken nicht besonders genau lokalisiert werden können; dies wäre zum Beispiel der Fall für besonders kleine anatomische Landmarke oder solche anatomischen Landmarken, die in den intraoperativen Bilddaten verdeckt oder nur teilweise sichtbar sind. In einem solchen Szenario könnte die Unsicherheit der Lokalisierung als besonders groß angenommen werden und entsprechende auch die Unsicherheit für die Alternative Schätzung der Patienten-Registrierung als entsprechend groß angenommen werden.

Auch die topographische Rekonstruktion aus Box 3240 kann mit einer bestimmten Unsicherheit behaftet sein und auf diese Unsicherheit kann verwendet werden, um die Unsicherheit der alternativen Schätzung der Patienten-Registrierung zu bestimmen. Beispielsweise können bestimmte Formen oder Texturen von anatomischen Landmarken besser für eine topographische Rekonstruktion geeignet sein als andere Formen oder Texturen. Außerdem wäre es denkbar, dass die Unsicherheit der alternativen Schätzung der Patienten-Registrierung ortsaufgelöst bestimmt wird: so könnte eine höhere bzw. geringere Unsicherheit der alternativen Schätzung der Patienten-Registrierung für größere bzw. kleinere Abstände von ein oder mehreren anatomischen Landmarken anhand derer die alternative Schätzung der Patienten-Registrierung bestimmt wird, angenommen werden.

Anschließend kann in Box 3255 überprüft werden, ob in der Liste der Kandidaten-Landmarke noch eine weitere Kandidaten-Landmarke beinhaltet ist. Ist dies der Fall, wird eine weitere Iteration 3291 von Box 3220 ausgeführt und eine weitere Kandidaten-Landmarke aus der Liste ausgewählt. Daraus ist ersichtlich, dass - sofern in der Liste mehrere Kandidaten-Landmarken beinhaltet sind - durch die mehreren Iterationen 3291 von Box 3250 mehrere Schätzungen für die Patienten-Registrierung oder die Unsicherheit erhalten werden. Entsprechend ist es nach Bearbeitung aller Kandidaten-Landmarken ("Nein"-Pfad aus Box 3255) in Box 3260 möglich, die entsprechenden Ergebnisse der mehreren Iterationen 3291 von Box 3250 zu fusionieren. Beispielsweise könnte ein Mittelwert gebildet werden. Dann kann die Kontextinformation basierend auf dem Ergebnis der Fusion bestimmt werden.

Anschließend kann das nächste Bild in einer weiteren Installation 3290 von Box 3215 erhalten werden.

Es sind verschiedene Modifikationen des Verfahrens aus FIG. 7 denkbar. Beispielsweise wurde in FIG. 7 ein Szenario gezeigt, bei denen in den Iterationen 3291 jemals individuell unterschiedliche Kandidaten-Landmarken abgearbeitet werden. Es wäre aber denkbar, dass beispielsweise zur Detektion in Box 3225 ein maschinengelerntes Modell verwendet wird, welches gemeinsam mehrere unterschiedliche Typen von Kandidaten-Landmarken detektieren kann. Das bedeutet dann in anderen Worten, dass anstatt der Auswahl einer einzigen Kandidaten-Landmarken Box 3220 beispielsweise mehrere Kandidaten-Landmarken in Box 3220 ausgewählt werden.

In einer weiteren Variante wäre es denkbar, dass die Lokalisierung und Detektion nicht in unterschiedlichen Boxen (Box 3225 und Box 3235) durchgeführt wird sondern in einer gemeinsamen Box. Beispielsweise könnte eine semantische Segmentierung durchgeführt werden, welche zwischen unterschiedlichen Typen von anatomischen Landmarken im Wege einer Klassifikation unterscheidet und gleichzeitig die erkannten anatomischen Landmarken segmentiert.

FIG. 8 ist ein Flussdiagramm eines beispielhaften Verfahrens. FIG. 8 illustriert ein Verfahren zur Nachverfolgung einer anatomischen Landmarke, die nacheinander in unterschiedlichen Bildern der intraoperativen Bilddaten aufgefunden wird. Das Verfahren aus FIG. 8 betrifft TAB. 1, Beispiel 2. Gemäß dem Verfahren aus FIG. 8 kann überprüft werden, ob eine geeignete Referenz-Position und/oder Referenz-Orientierung für die jeweilige Landmarke verfügbar ist.

Beispielsweise könnte das Verfahren der FIG. 8 in jeder Iteration 3290 für ein oder mehrere der Kandidaten-Landmarken ausgeführt werden. Wenn eine Landmarke eines bestimmten Typs in dem jeweiligen Bild aufgefunden und lokalisiert wird (vergleiche zum Beispiel FIG. 7: Box 3230 und Box 3235), so kann diese anschließend in Box 3510 charakterisiert werden. Die Landmarke wird anhand ihres Erscheinungsbilds und/oder der Position innerhalb der Bilddaten charakterisiert. Beispielsweise kann eine charakteristische Form oder Textur der Landmarke ermittelt werden.

Es ist dann in Box 3515 möglich, solche Information zur Charakterisierung der anatomischen Landmarke mit früher ermittelter Information zur Charakterisierung von früher beobachteten anatomischen Landmarken (das heißt in früheren Iterationen 3290, vergleiche FIG. 7) zu vergleichen. Derart kann bestimmt werden, ob eine bestimmte anatomischen Landmarke bereits zu einem früheren Zeitpunkt beobachtet wurde.

Wenn die Landmarke bereits bekannt ist, so kann optional in Box 3516 bestimmt werden, ob eine geeignete Referenz-Position und/oder Referenz-Orientierung für diese bereits bekannte Landmarke verfügbar ist. Beispielsweise könnte ein zeitlicher Filter angewendet werden.

Im Detail ist die gegenwärtige Position und/oder Orientierung der Landmarke mit einem ersten Zeitpunkt assoziiert; und die Referenz-Position und/oder Referenz-Orientierung der Landmarke ist mit einem zweiten Zeitpunkt assoziiert. Es könnte im Rahmen der zeitlichen Filterung gefordert werden, dass die Zeitspanne zwischen dem ersten Zeitpunkt und dem zweiten Zeitpunkt bestimmte Kriterien erfüllt, das heißt zum Beispiel größer als ein unterer Schwellenwert ist oder kleiner als ein oberer Schwellenwert ist. Beispielsweise könnte (wie im Zusammenhang mit FIG. 6 gezeigt) ein Vergleich der Position und/oder Orientierung der Landmarke zu Beginn des Beobachtungszeitraums 69 mit der Position und/oder Orientierung der Landmarke zum Ende des Beobachtungszeitraums 69 durchgeführt werden. Der Beobachtungszeitraum 69 könnte gemäß einem Schiebefensterverfahren bestimmt werden, das heißt kontinuierlich mit Erhalten weiterer Bilder im Zeitraum vorgeschoben werden. Es wäre aber auch möglich, dass der Beobachtungszeitraum immer bei der initialen Bestimmung der Patienten-Registrierung beginnt. Die Länge des Beobachtungszeitraums 69 könnte zum Beispiel in Abhängigkeit von einem Typ des operativen Eingriffs bestimmt werden. Beispielsweise kann es vorkommen, dass unterschiedliche Typen von operativen Eingriffen einen unterschiedlichen Grad an Dynamik aufweisen; so dass es hilfreich ist, diese unterschiedlichen erwarteten Geschwindigkeiten bei der Änderung der beobachteten Szene auch durch die daran angepasste Länge des Beobachtungszeitraums 69 zu überwachen. Entsprechend wäre es denkbar, keinen Vergleich in Box 3520 durchzuführen, wenn die Landmarke zwar grundsätzlich bekannt ist; aber für einen solch langen Zeitraum in den intraoperativen Bilddaten nicht sichtbar gewesen ist, dass die letzte bekannte Referenz-Position und/oder Referenz-Orientierung nicht mehr aktuell ist.

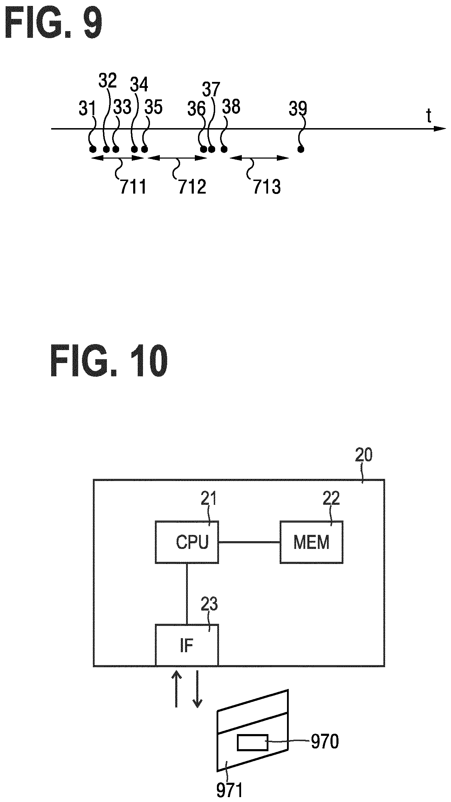

In jedem Fall kann in Box 3525 die aktuelle Position und/oder Orientierung der anatomischen Landmarke gespeichert werden. Gegebenenfalls kann die Speicherung zusammen mit einem Zeitstempel erfolgen, so dass, wie obenstehend beschrieben, auch eine zeitliche Filterung durchgeführt werden kann. Es kann auch eine eindeutige ID für jede erkannte Landmarke vergeben werden. Das Verfahren aus FIG. 8 ermöglicht es, den Vergleich der Position und/oder der Orientierung der in den intraoperativen Bilddaten gefundenen anatomischen Landmarke mit einer früher beobachteten Referenz-Position und/oder Referenz-Orientierung derselben anatomischen Landmarke durchzuführen - selbst wenn die anatomische Landmarke während Teilen des entsprechenden Beobachtungszeitraums in den intraoperativen Bilddaten nicht sichtbar gewesen war. Dies ist auch im Zusammenhang mit FIG. 9 dargestellt.

In FIG. 9 ist eine Abfolge von Bildern 31-39 in den intraoperativen Bilddaten dargestellt, in denen die anatomische Landmarke aufgefunden wird und lokalisiert werden kann. Im Detail liegen in einem Zeitintervall 711 die Bilder 31-35. Alle Bilder 31-35 zeigen die anatomische Landmarke, so dass dort die anatomische Landmarke aufgefunden und lokalisiert werden kann. Aus FIG. 9 ist ersichtlich, dass zum Beispiel im Zeitintervall 712 sowie anschließend im Zeitintervall 713 die anatomische Landmarke nicht in den Bildern der intraoperativen Bilddaten aufgefunden werden kann. Allerdings ist die anatomische Landmarke zwischenzeitlich in den Bildern 36, 37, 38 sichtbar. Beispielsweise wäre es denkbar, dass zu jedem Bild 31-39 in dem die anatomische Landmarke gefunden und lokalisiert werden kann die Genauigkeit bzw. Unsicherheit im Zusammenhang mit der daraus ermittelten Kontextinformation (beispielsweise eine alternative Schätzung für die Patienten-Registrierung) ermittelt wird. Beispielsweise wäre es denkbar, dass eine solche Unsicherheit für eine alternative Schätzung der Patienten-Registrierung umso geringer angenommen wird, je länger das durchgängige Zeitintervall (beispielsweise das Zeitintervall 711) während dessen die anatomische Landmarke wiederholt gefunden und lokalisiert werden kann.

Insbesondere wäre es denkbar, dass in den Bildern 31-35, die in dem Zeitintervall 711 liegen, ein Filter angewendet wird, bei dem beispielsweise die Lokalisierung der Landmarke in einem Bild 34 auch in Abhängigkeit von der Lokalisierung der Landmarken den vorherigen Bildern 31-33 abhängt. Derart kann die Zuverlässigkeit für die Lokalisierung typischerweise erhöht werden. Wenn dann für eine bestimmte Mindestzeitdauer (vergleiche Zeitintervall 712) die anatomische Landmarke verdeckt ist, könnte dieser Filter neu initialisiert werden.

FIG. 10 illustriert schematisch eine elektronische Datenverarbeitungsvorrichtung 20 für ein chirurgisches Assistenzsystem. Die Datenverarbeitungsvorrichtung umfasst einen Prozessor 21 sowie einen Speicher 22 und eine Kommunikationsschnittstelle 23. Der Prozessor 21 kann Programmcode aus dem Speicher 22 laden und ausführen. In den verschiedenen hierin beschriebenen Beispielen kann die Datenverarbeitung auch in der Cloud geschehen. Es werden auch kombinierte Cloud-On Premise-Implementierungen denkbar. Beispielsweise könnte der Speicher 22 zumindest teilweise in der Cloud sein. Wenn der Prozessor 21 den Programmcode ausführt, bewirkt dies, dass der Prozessor 21 Techniken, wie sie voranstehend beschrieben sind, ausführt. Beispielsweise könnte der Prozessor 21 Bilddaten über die Kommunikationsschnittstelle 23 empfangen, die Bilddaten auswerten, um ein oder mehrere anatomische Landmarken zu lokalisieren, Koordinatentransformationen basierend auf ein oder mehreren Registrierungen durchführen, Kontextinformation für eine Schätzung einer Patientenregistrierung bestimmen, ein Steuersignal basierend auf der Kontextinformation zu bestimmen und optional zu senden, beispielsweise indem ein Datenrahmen mit der Kontextinformation bestimmt wird und über die Kommunikationsschnittstelle 23 gesendet wird, usw. Beispielsweise ist in FIG. 10 dargestellt, wie das Steuersignal 971 über die Kommunikationsschnittstelle 23 an eine oder mehrere Komponenten, die mit dem chirurgischen Assistenzsystem assoziiert sind, gesendet wird. Das Steuersignal 971 umfasst dabei die Kontextinformation 970. Grundsätzlich wäre es denkbar, dass unterschiedliche Steuersignalen unterschiedliche Komponenten gesendet werden. In einem solchen Fall ist es denkbar, dass unterschiedliche Steuersignalen unterschiedliche Teile der Kontextinformation 970 beinhalten. So wäre es beispielsweise denkbar, dass eine erstes Steuersignal an das Navigationssystem gesendet wird. Dieses Steuersignal kann beispielsweise indikativ für eine Unsicherheit oder eine alternative Schätzung für die Patienten-Registrierung sein. Das Navigationssystem kann dieses Steuersignal empfangen und basierend darauf die eigene Schätzung der Patienten-Registrierung anpassen. Entsprechende Techniken wurden voranstehend im Zusammenhang mit FIG. 4, Pfeil von Box 3170 zu Box 3110 diskutiert. Ein zweites Steuersignal kann an eine grafische Benutzerschnittstelle gesendet werden. Dieses zweite Steuersignal kann eine basierend auf der Unsicherheit der Patienten-Registrierung ermittelte Warnung beinhalten und die Benutzerschnittstelle auffordern, die Warnung an den Patienten auszugeben. Als allgemeine Regel kann ein chirurgisches Assistenzsystem, welches die hierin beschriebenen Techniken ausführt, mehrere Prozessoren und auch mehrere elektronische Datenverarbeitungsvorrichtungen wie beispielsweise die elektronische Datenverarbeitungsvorrichtung 20 aus FIG. 10 umfassen. Beispielsweise wäre es denkbar, dass Teile der Logik auf einer elektronischen Datenverarbeitungsvorrichtung eines Navigationssystems (vergleiche FIG. 1: Navigationssystem 899) ausgeführt werden und andere Teile der Logik auf einer elektronischen Datenverarbeitungsvorrichtung eines Operationsmikroskops (vergleiche FIG. 1: Operationsmikroskop 802). Beispielsweise wäre es insbesondere möglich, dass eine erste elektronische Datenverarbeitungsvorrichtung eine Schätzung der Patienten-Registrierung basierend auf einem Marker-Koordinatensystem, welches als Proxy für das Patienten-Koordinatensystem dient, bestimmt. Beispielsweise könnte die erste elektronische

Datenverarbeitungsvorrichtung den iterativen Prozess 3001 aus FIG. 4 ausführen. Eine davon getrennte, zweite elektronische Datenverarbeitungsvorrichtung könnte die Kontextinformation für die Schätzung der Patienten-Registrierung und das Steuersignal bestimmen. Beispielsweise könnte die zweite elektronische Datenverarbeitungsvorrichtung den iterativen Prozess 3002 aus FIG. 4 ausführen. Beispielsweise könnte die erste elektronische Datenverarbeitungsvorrichtung Teil eines Navigationssystems sein, welches das globale Referenz-Koordinatensystem definiert; während die zweite elektronische Datenverarbeitungsvorrichtung Teil eines Operationsmikroskops sein könnte.

Zusammenfassend wurden insbesondere die folgenden Beispiele der ersten Gruppe offenbart und beschrieben:
Beispiel 1. Chirurgisches Assistenzsystem (20, 802, 899) umfassend mindestens einen Prozessor (21) und mindestens einen Speicher (22), wobei der mindestens eine Prozessor (21) eingerichtet ist, um Programmcode aus dem mindestens einen Speicher (22) zu laden und auszuführen, wobei der mindestens eine Prozessor (21) basierend auf dem Ausführen des Programmcodes die folgenden Schritte ausführt:
   - basierend auf Referenzmarkern (892), die lagefest an einer an einem Skelett eines Patienten angebrachten Halterung (891) befestigt sind: Nachverfolgen (3105) einer Lage der Halterung (891) während eines Beobachtungszeitraums (69) in einem Referenz-Koordinatensystem (91),
      - basierend auf der Nachverfolgung der Lage der Halterung (891), Bestimmen (3110) einer Schätzung einer Patienten-Registrierung (85) im Referenz-Koordinatensystem (91) während des Beobachtungszeitraums (69),
      - Erhalten von intraoperativen Bilddaten, welche während des Beobachtungszeitraums (69) mittels eines chirurgischen Visualisierungssystems (802, 809) erfasst sind, wobei das chirurgische Visualisierungssystem in Bezug auf den Patienten beweglich ist,
      - basierend auf den intraoperativen Bilddaten, Bestimmen einer Schätzung einer Position und/oder Orientierung einer in den intraoperativen Bilddaten dargestellten anatomischen Landmarke (75) in dem Referenz-Koordinatensystem (91), wobei die anatomische Landmarke (75) in Bezug auf das Skelett lagefest ist,
      - Durchführen eines Vergleichs der Schätzung der Position und/oder Orientierung der anatomischen Landmarke (75) mit einer vorgegebenen Referenz-Position und/oder Referenz-Orientierung, die aus Referenz-Bilddaten (60) des Patienten und anhand der Schätzung der Patienten-Registrierung (85) bestimmt ist,
      - basierend auf einem Ergebnis des Vergleichs, Bestimmen von Kontextinformation für die Schätzung der Patienten-Registrierung (85), und
      - basierend auf der Kontextinformation, Erzeugen eines Steuersignals für ein oder mehrere mit dem chirurgischen Assistenzsystem assoziierte Komponenten.
Beispiel 2. Chirurgisches Assistenzsystem nach Beispiel 1,
   wobei die anatomische Landmarke (75) basierend auf einer vordefinierten Liste mit erlaubten und/oder verbotenen Kandidaten-Landmarken aufgefunden wird.
Beispiel 3. Chirurgisches Assistenzsystem nach Beispiel 2,
   wobei die vordefinierte Liste basierend auf einem Typ des operativen Eingriffs bestimmt ist.
Beispiel 4. Chirurgisches Assistenzsystem nach Beispiel 3,
   wobei zum Bestimmen der vordefinierten Liste verwendete Typinformation, welche indikativ für den Typ des operativen Eingriffs ist, mehrere Hierarchieebenen umfasst.
Beispiel 5. Chirurgisches Assistenzsystem nach Beispiel 4,
   wobei eine erste Hierarchieebene der mehreren Hierarchieebenen eine Eingriffsregion des jeweiligen Typs des operativen Eingriffs spezifiziert,
   wobei eine zweite Hierarchieebene der mehreren Hierarchieebenen eine Orientierung eines Zugangskanals innerhalb der Eingriffsregion für den jeweiligen Typ des operativen Eingriffs spezifiziert.
Beispiel 6. Chirurgisches Assistenzsystem nach einem der Beispiele 2 bis 5,
   wobei die vordefinierte Liste eine Priorisierung für die erlaubten und/oder verbotenen Kandidaten-Landmarken für einen jeweiligen Typ des operativen Eingriffs beinhaltet.
Beispiel 7. Chirurgisches Assistenzsystem nach einem der Beispiele 2 bis 6,
   wobei der mindestens eine Prozessor (21) basierend auf dem Ausführen des Programmcodes weiterhin den folgenden Schritt ausführt:
   - Überprüfen (3211), ob die Anzahl der in der vordefinierten Liste vorhandenen erlaubten Kandidaten-Landmarken einen vorgegebenen Schwellenwert überschreitet, und
      - wenn die Anzahl der erlaubten Kandidaten-Landmarken den vorgegebenen Schwellenwert nicht überschreitet: Ansteuern (3212) einer Benutzerschnittstelle mittels des Steuersignals zum Ausgeben einer Warnung.
Beispiel 8. Chirurgisches Assistenzsystem nach einem der voranstehenden Beispiele,
   wobei die anatomische Landmarke (75) während Teilen des Beobachtungszeitraums (69) nicht in den intraoperativen Bilddaten sichtbar ist.
Beispiel 9. Chirurgisches Assistenzsystem nach eine der voranstehenden Beispiele,
   wobei der mindestens eine Prozessor (21) basierend auf dem Ausführen des Programmcodes weiterhin zumindest einen der folgenden Schritte ausführt: eine Detektion (3225) der anatomischen Landmarke in den intraoperativen Bilddaten, eine Lokalisierung (3235) in den intraoperativen Bilddaten basierend auf Vorwissen über ein Erscheinungsbild der anatomischen Landmarke, ein Tracking ein oder mehrerer anatomischer Landmarken über der Zeit, einer topographischen Rekonstruktion (3240) einer Oberfläche der anatomischen Landmarke, ein Posen-Schätzungsmodell zum Bestimmen einer Form und Lage der anatomischen Landmarke, und eine Umrechnung (3245) der Position und/oder Orientierung der anatomischen Landmarke in das Referenz-Koordinatensystem oder ein weiteres Referenz-Koordinatensystem.
Beispiel 10. Chirurgisches Assistenzsystem nach einem der voranstehenden Beispiele,
   wobei der mindestens eine Prozessor (21) weiterhin eingerichtet ist, um basierend auf dem Ausführen des Programmcodes den folgenden Schritt auszuführen:
   - Einstellen eines Schwellenwerts (219) für den Vergleich in Abhängigkeit von ein oder mehreren Kriterien.
Beispiel 11. Chirurgisches Assistenzsystem nach Beispiel 10,
   wobei die ein oder mehreren Kriterien einen Typ des operativen Eingriffs umfassen.
Beispiel 12. Chirurgisches Assistenzsystem nach Beispiel 10 oder 11,
   wobei die ein oder mehreren Kriterien eine Vorhersage für einen gegenwärtigen oder nachfolgenden Operationsschritt des operativen Eingriffs umfassen.
Beispiel 13. Chirurgisches Assistenzsystem nach einem der voranstehenden Beispiele,
   wobei die Kontextinformation ortsaufgelöst für einer Umgebung der anatomischen Landmarke (75) und/oder anhand von Szeneninformation bestimmt wird.
Beispiel 14. Chirurgisches Assistenzsystem nach einem der voranstehenden Beispiele,
   wobei der mindestens eine Prozessor (21) weiterhin eingerichtet ist, um basierend auf dem Ausführen des Programmcodes den folgenden Schritt auszuführen:
   - basierend auf der Kontextinformation: Ansteuern einer Benutzerschnittstelle zum Indizieren von lokalen Bereichen mit besonders hoher oder niedriger Konfidenz für die Schätzung der Patienten-Registrierung (85).
Beispiel 15. Chirurgisches Assistenzsystem nach einem der voranstehenden Beispiele,
   wobei die Kontextinformation eine alternative Schätzung der Patienten-Registrierung (85) umfasst.
Beispiel 16. Chirurgisches Assistenzsystem nach einem der voranstehenden Beispiele,
   wobei die Kontextinformation eine Unsicherheit für die Schätzung der Patienten-Registrierung (85) umfasst.
Beispiel 17. Chirurgisches Assistenzsystem nach eine der voranstehenden Beispiele,
   wobei der mindestens eine Prozessor (21) basierend auf dem Ausführen des Programmcodes weiterhin den folgenden Schritt ausführt:
   - Ansteuern eines Navigationssystem basierend auf dem Steuersignal.
Beispiel 18. Chirurgisches Assistenzsystem nach eine der voranstehenden Beispiele,
   wobei der mindestens eine Prozessor (21) basierend auf dem Ausführen des Programmcodes weiterhin den folgenden Schritt ausführt:
   - Suchen ein oder mehrerer vorgegebener Kandidaten-Landmarken in den intraoperativen Bilddaten, und
   - wenn das Suchen der ein oder mehreren vorgegebenen Kandidaten-Landmarken für eine vorgegebenen Zeitdauer erfolglos verbleibt, Anfordern einer Erweiterung eines Gesichtsfelds des chirurgischen Visualisierungssystems.
Beispiel 19. Computer-implementiertes Verfahren, das umfasst:
   - basierend auf Referenzmarkern (892), die lagefest an einer an einem Skelett eines Patienten angebrachten Halterung (891) befestigt sind: Nachverfolgen (3105) einer Lage der Halterung (891) während eines Beobachtungszeitraums (69) in einem Referenz-Koordinatensystem (91),
      - basierend auf der Nachverfolgung der Lage der Halterung (891), Bestimmen (3110) einer Schätzung einer Patienten-Registrierung (85) im Referenz-Koordinatensystem (91) während des Beobachtungszeitraums (69),
      - Erhalten von intraoperativen Bilddaten, welche während des Beobachtungszeitraums (69) mittels eines chirurgischen Visualisierungssystems (802, 809) erfasst sind, wobei das chirurgischen Visualisierungssystem in Bezug auf den Patienten beweglich ist,
      - basierend auf den intraoperativen Bilddaten, Bestimmen einer Schätzung einer Position und/oder Orientierung einer in den intraoperativen Bilddaten dargestellten anatomischen Landmarke (75) in dem Referenz-Koordinatensystem (91), wobei die anatomische Landmarke (75) in Bezug auf das Skelett lagefest ist,
   - Durchführen eines Vergleichs der Schätzung der Position und/oder Orientierung der anatomischen Landmarke (75) mit einer vorgegebenen Referenz-Position und/oder Referenz-Orientierung, die aus Referenz-Bilddaten (60) des Patienten und anhand der Schätzung der Patienten-Registrierung (85) bestimmt ist,
   - basierend auf einem Ergebnis des Vergleichs, Bestimmen von Kontextinformation für die Schätzung der Patienten-Registrierung (85), und
   - basierend auf der Kontextinformation, Erzeugen eines Steuersignals für ein oder mehrere mit einem chirurgischen Assistenzsystem assoziierte Komponenten.
Beispiel 20. Computer-implementiertes Verfahren nach Beispiel 19,
   wobei das Verfahren von dem chirurgischen Assistenzsystem nach einem der Beispiele 1 bis 18 ausgeführt wird.
Beispiel 21. Elektronische Datenverarbeitungsvorrichtung umfassend mindestens einen Prozessor (21) und mindestens einen Speicher (22), wobei der mindestens eine Prozessor (21) eingerichtet ist, um Programmcode aus dem mindestens einen Speicher (22) zu laden und auszuführen, wobei der mindestens eine Prozessor (21) basierend auf dem Ausführen des Programmcodes die folgenden Schritte ausführt:
   - Erhalten von intraoperativen Bilddaten, welche während eines Beobachtungszeitraums (69) mittels eines chirurgischen Visualisierungssystems (802, 809) erfasst sind, wobei das chirurgischen Visualisierungssystem in Bezug auf einen Patienten beweglich ist,
   - basierend auf den intraoperativen Bilddaten, Bestimmen einer Schätzung einer Position und/oder Orientierung einer in den intraoperativen Bilddaten dargestellten anatomischen Landmarke (75) in einem Referenz-Koordinatensystem (91), wobei die anatomische Landmarke (75) in Bezug auf das Skelett lagefest ist,
   - Durchführen eines Vergleichs der Schätzung der Position und/oder Orientierung der anatomischen Landmarke (75) mit einer vorgegebenen Referenz-Position und/oder Referenz-Orientierung, die aus Referenz-Bilddaten (60) des Patienten und anhand einer Schätzung der Patienten-Registrierung (85) bestimmt ist,
   - basierend auf einem Ergebnis des Vergleichs, Bestimmen von Kontextinformation für die Schätzung der Patienten-Registrierung (85), und
   - basierend auf der Kontextinformation, Erzeugen eines Steuersignals für ein oder mehrere mit dem chirurgischen Assistenzsystem assoziierte Komponenten.
Beispiel 22. Elektronische Datenverarbeitungsvorrichtung für ein chirurgisches Navigationssystem (899), umfassend mindestens einen Prozessor (21) und mindestens einen Speicher (22), wobei der mindestens eine Prozessor (21) eingerichtet ist, um Programmcode aus dem mindestens einen Speicher (22) zu laden und auszuführen, wobei der mindestens eine Prozessor (21) basierend auf dem Ausführen des Programmcodes die folgenden Schritte ausführt:
   - basierend auf Referenzmarkern (892), die lagefest an einer an einem Skelett eines Patienten angebrachten Halterung (891) befestigt sind: Nachverfolgen (3105) einer Lage der Halterung (891) während eines Beobachtungszeitraums (69) in einem Referenz-Koordinatensystem (91),
      - basierend auf der Nachverfolgung der Lage der Halterung (891), Bestimmen (3110) einer Schätzung einer Patienten-Registrierung (85) im Referenz-Koordinatensystem (91) während des Beobachtungszeitraums (69),
      - Empfangen eines Steuersignals, welches Kontextinformation für die Schätzung der Patienten-Registrierung indiziert,
      - basierend auf der Kontextinformation, Anpassen der Schätzung der Patienten-Registrierung.
Beispiel 23. System, umfassend die elektronische Datenverarbeitungsvorrichtung nach Beispiel 21 sowie die elektronische Datenverarbeitungsvorrichtung nach Beispiel 22.
Beispiel 24. Computer-implementiertes Verfahren, umfassend:
   - Erhalten von intraoperativen Bilddaten, welche während eines Beobachtungszeitraums (69) mittels eines chirurgischen Visualisierungssystems (802, 809) erfasst sind, wobei das chirurgischen Visualisierungssystem in Bezug auf einen Patienten beweglich ist,
   - basierend auf den intraoperativen Bilddaten, Bestimmen einer Schätzung einer Position und/oder Orientierung einer in den intraoperativen Bilddaten dargestellten anatomischen Landmarke (75) in einem Referenz-Koordinatensystem (91), wobei die anatomische Landmarke (75) in Bezug auf das Skelett lagefest ist,
   - Durchführen eines Vergleichs der Schätzung der Position und/oder Orientierung der anatomischen Landmarke (75) mit einer vorgegebenen Referenz-Position und/oder Referenz-Orientierung, die aus Referenz-Bilddaten (60) des Patienten und anhand einer Schätzung der Patienten-Registrierung (85) bestimmt ist,
   - basierend auf einem Ergebnis des Vergleichs, Bestimmen von Kontextinformation für die Schätzung der Patienten-Registrierung (85), und
   - basierend auf der Kontextinformation, Erzeugen eines Steuersignals für ein oder mehrere mit dem chirurgischen Assistenzsystem assoziierte Komponenten.

Außerdem wurden auch die folgenden Beispiele der zweiten Gruppe beschrieben (wobei die Beispiele der ersten Gruppe und der zweiten Gruppe miteinander kombiniert werden können).

Beispiel 1. Chirurgisches Assistenzsystem (20, 802, 899) umfassend mindestens einen Prozessor (21) und mindestens einen Speicher (22), wobei der mindestens eine Prozessor (21) eingerichtet ist, um Programmcode aus dem mindestens einen Speicher (22) zu laden und auszuführen, wobei der mindestens eine Prozessor (21) basierend auf dem Ausführen des Programmcodes die folgenden Schritte ausführt:
- basierend auf Referenzmarkern (892), die lagefest an einer an einem Skelett eines Patienten angebrachten Halterung (891) befestigt sind: Nachverfolgen (3105) einer Lage der Halterung (891) während eines Beobachtungszeitraums (69) in einem Referenz-Koordinatensystem (91),
- basierend auf der Nachverfolgung der Lage der Halterung (891), Bestimmen (3110) einer Schätzung einer Patienten-Registrierung (85) im Referenz-Koordinatensystem (91) während des Beobachtungszeitraums (69),
- Erhalten von intraoperativen Bilddaten, welche während des Beobachtungszeitraums (69) mittels eines chirurgischen Visualisierungssystems (802, 809) erfasst sind, wobei das chirurgische Visualisierungssystem in Bezug auf den Patienten beweglich ist,
- basierend auf den intraoperativen Bilddaten, Bestimmen einer Position und/oder Orientierung einer in den intraoperativen Bilddaten dargestellten anatomischen Landmarke (75), wobei die anatomische Landmarke (75) in Bezug auf das Skelett lagefest ist,
- Durchführen eines Vergleichs, welcher eine Änderung der Schätzung der Patienten-Registrierung (85) während des Beobachtungszeitraums (69) in Bezug setzt zu einer Änderung der Position und/oder Orientierung der anatomischen Landmarke (75) während des Beobachtungszeitraums (69),
- basierend auf einem Ergebnis des Vergleichs, Bestimmen von Kontextinformation für die Schätzung der Patienten-Registrierung (85), und
- basierend auf der Kontextinformation, Erzeugen eines Steuersignals für ein oder mehrere mit dem chirurgischen Assistenzsystem assoziierte Komponenten.

Beispiel 2. Chirurgisches Assistenzsystem nach Beispiel 1,
wobei die anatomische Landmarke basierend auf einer vordefinierten Liste mit erlaubten und/oder verbotenen Kandidaten-Landmarken aufgefunden wird.

Beispiel 3. Chirurgisches Assistenzsystem nach Beispiel 2,
wobei die vordefinierte Liste basierend auf einem Typ des operativen Eingriffs bestimmt ist.

Beispiel 4. Chirurgisches Assistenzsystem nach Beispiel 3,
wobei zum Bestimmen der vordefinierten Liste verwendete Typinformation, welche indikativ für den Typ des operativen Eingriffs ist, mehrere Hierarchieebenen umfasst.

Beispiel 5. Chirurgisches Assistenzsystem nach Beispiel 4,
wobei eine erste Hierarchieebene der mehreren Hierarchieebenen eine Eingriffsregion des jeweiligen Typs des operativen Eingriffs spezifiziert,
wobei eine zweite Hierarchieebene der mehreren Hierarchieebenen eine Orientierung eines Zugangskanals innerhalb der Eingriffsregion für den jeweiligen Typ des operativen Eingriffs spezifiziert.

Beispiel 6. Chirurgisches Assistenzsystem nach einem der Beispiele 2 bis 5,
wobei die vordefinierte Liste eine Priorisierung für die erlaubten und/oder verbotenen Kandidaten-Landmarken beinhaltet.

Beispiel 7. Chirurgisches Assistenzsystem nach einem der Beispiele 2 bis 6,
wobei der mindestens eine Prozessor (21) basierend auf dem Ausführen des Programmcodes weiterhin den folgenden Schritt ausführt:
- Überprüfen (3211), ob die Anzahl der in der vordefinierten Liste vorhandenen erlaubten Kandidaten-Landmarken einen vorgegebenen Schwellenwert überschreitet, und
   - wenn die Anzahl der erlaubten Kandidaten-Landmarken den vorgegebenen Schwellenwert nicht überschreitet: Ansteuern (3212) einer Benutzerschnittstelle zum Ausgeben einer Warnung.

Beispiel 8. Chirurgisches Assistenzsystem nach eine der voranstehenden Beispiele,
wobei die anatomische Landmarke während Teilen des Beobachtungszeitraums (69) nicht in den intraoperativen Bilddaten sichtbar ist.

Beispiel 9. Chirurgisches Assistenzsystem nach einem der voranstehenden Beispiele,
wobei der mindestens eine Prozessor (21) basierend auf dem Ausführen des Programmcodes weiterhin zumindest einen der folgenden Schritte ausführt: eine Detektion (3225) der anatomischen Landmarke in den intraoperativen Bilddaten, eine Lokalisierung (3235) in den intraoperativen Bilddaten basierend auf Vorwissen über ein Erscheinungsbild der anatomischen Landmarke, ein Tracking ein oder mehrerer anatomischer Landmarken über der Zeit, einer topographischen Rekonstruktion (3240) einer Oberfläche der anatomischen Landmarke, ein Posen-Schätzungsmodell zum Bestimmen einer Form und Lage der anatomischen Landmarke, und eine Umrechnung (3245) der Position und/oder Orientierung der anatomischen Landmarke in das Referenz-Koordinatensystem oder ein weiteres Referenz-Koordinatensystem.

Beispiel 10. Chirurgisches Assistenzsystem nach einem der voranstehenden Beispiele,
wobei der mindestens eine Prozessor (21) weiterhin eingerichtet ist, um basierend auf dem Ausführen des Programmcodes den folgenden Schritt auszuführen:
- Einstellen eines Grenzwerts für den Vergleich in Abhängigkeit von ein oder mehreren Kriterien.

Beispiel 11. Chirurgisches Assistenzsystem nach Beispiel 10,
wobei die ein oder mehreren Kriterien einen Typ des operativen Eingriffs umfassen.

Beispiel 12. Chirurgisches Assistenzsystem nach Beispiel 10 oder 11,
wobei die ein oder mehreren Kriterien eine Vorhersage für einen gegenwärtigen oder nachfolgenden Operationsschritt des operativen Eingriffs umfassen.

Beispiel 13. Chirurgisches Assistenzsystem nach einem der voranstehenden Beispiele,
wobei die Kontextinformation ortsaufgelöst für eine Umgebung der anatomischen Landmarke (75) und/oder anhand von Szeneninformation bestimmt wird.

Beispiel 14. Chirurgisches Assistenzsystem nach einem der voranstehenden Beispiele,
wobei der mindestens eine Prozessor (21) weiterhin eingerichtet ist, um basierend auf dem Ausführen des Programmcodes den folgenden Schritt auszuführen:
- basierend auf der Kontextinformation: Ansteuern einer Benutzerschnittstelle mittels des Steuersignals zum Indizieren von lokalen Bereichen mit besonders hoher oder niedriger Konfidenz für die Schätzung der Patienten-Registrierung (85).

Beispiel 15. Chirurgisches Assistenzsystem nach einem der voranstehenden Beispiele,
wobei die Kontextinformation eine alternative Schätzung der Patienten-Registrierung (85) umfasst.

Beispiel 16. Chirurgisches Assistenzsystem nach einem der voranstehenden Beispiele,
wobei die Kontextinformation eine Unsicherheit für die Schätzung der Patienten-Registrierung (85) umfasst.

Beispiel 17. Chirurgisches Assistenzsystem nach eine der voranstehenden Beispiele,
wobei der mindestens eine Prozessor (21) basierend auf dem Ausführen des Programmcodes weiterhin den folgenden Schritt ausführt:
- Ansteuern eines Navigationssystem basierend auf dem Steuersignal.

Beispiel 18. Chirurgisches Assistenzsystem nach eine der voranstehenden Beispiele,
wobei der mindestens eine Prozessor (21) basierend auf dem Ausführen des Programmcodes weiterhin den folgenden Schritt ausführt:
- Suchen ein oder mehrerer vorgegebener Kandidaten-Landmarken in den intraoperativen Bilddaten, und
- wenn das Suchen der ein oder mehreren vorgegebenen Kandidaten-Landmarken für eine vorgegebenen Zeitdauer erfolglos verbleibt, Anfordern (3231) einer Erweiterung eines Gesichtsfelds des chirurgischen Visualisierungssystems.

Beispiel 19. Chirurgisches Assistenzsystem nach einem der voranstehenden Beispiele,
wobei der Beobachtungszeitraum (69) gemäß einem Schiebefensterverfahren bestimmt ist.

Beispiel 20. Chirurgisches Assistenzsystem nach einem der voranstehenden Beispiele,
wobei eine Länge des Beobachtungszeitraums (69) in Abhängigkeit von einem Typ des operativen Eingriffs bestimmt ist.

Beispiel 21. Chirurgisches Assistenzsystem nach einem der voranstehenden Beispiele,
wobei der Vergleich abgebrochen oder pausiert wird, wenn die anatomischen Landmarke für einen vorgegebenen Zeitraum in den intraoperativen Bilddaten nicht sichtbar ist.

Beispiel 22. Computer-implementiertes Verfahren, das umfasst:
- basierend auf Referenzmarkern (892), die lagefest an einer an einem Skelett eines Patienten angebrachten Halterung (891) befestigt sind: Nachverfolgen (3105) einer Lage der Halterung (891) während eines Beobachtungszeitraums (69) in einem Referenz-Koordinatensystem (91),
- basierend auf der Nachverfolgung der Lage der Halterung (891), Bestimmen (3110) einer Schätzung einer Patienten-Registrierung (85) im Referenz-Koordinatensystem (91) während des Beobachtungszeitraums (69),
- Erhalten von intraoperativen Bilddaten, welche während des Beobachtungszeitraums (69) mittels eines chirurgischen Visualisierungssystems (802, 809) erfasst sind, wobei das chirurgischen Visualisierungssystem in Bezug auf den Patienten beweglich ist,
- basierend auf den intraoperativen Bilddaten, Bestimmen einer Position und/oder Orientierung einer in den intraoperativen Bilddaten dargestellten anatomischen Landmarke (75), wobei die anatomische Landmarke (75) in Bezug auf das Skelett lagefest ist,
- Durchführen eines Vergleichs, welcher eine Änderung der Schätzung der Patienten-Registrierung (85) während des Beobachtungszeitraums (69) in Bezug setzt zu einer Änderung der Position und/oder Orientierung der anatomischen Landmarke (75) während des Beobachtungszeitraums (69),
- basierend auf einem Ergebnis des Vergleichs, Bestimmen von Kontextinformation für die Schätzung der Patienten-Registrierung (85), und
- basierend auf der Kontextinformation, Erzeugen eines Steuersignals für ein oder mehrere mit einem chirurgischen Assistenzsystem assoziierte Komponenten.

Beispiel 23. Computer-implementiertes Verfahren nach Beispiel 22,
wobei das Verfahren von dem chirurgischen Assistenzsystem nach einem der Beispiele 1 bis 21 ausgeführt wird.

Beispiel 24. Elektronische Datenverarbeitungsvorrichtung umfassend mindestens einen Prozessor (21) und mindestens einen Speicher (22), wobei der mindestens eine Prozessor (21) eingerichtet ist, um Programmcode aus dem mindestens einen Speicher (22) zu laden und auszuführen, wobei der mindestens eine Prozessor (21) basierend auf dem Ausführen des Programmcodes die folgenden Schritte ausführt:
- Erhalten von intraoperativen Bilddaten, welche während eines Beobachtungszeitraums (69) mittels eines chirurgischen Visualisierungssystems (802, 809) erfasst sind, wobei das chirurgischen Visualisierungssystem in Bezug auf einen Patienten beweglich ist,
- basierend auf den intraoperativen Bilddaten, Bestimmen einer Position und/oder Orientierung einer in den intraoperativen Bilddaten dargestellten anatomischen Landmarke (75), wobei die anatomische Landmarke (75) in Bezug auf das Skelett lagefest ist,
- Durchführen eines Vergleichs, welcher eine Änderung einer Schätzung der Patienten-Registrierung (85) während des Beobachtungszeitraums (69) in Bezug setzt zu einer Änderung der Position und/oder Orientierung der anatomischen Landmarke (75) während des Beobachtungszeitraums (69),
- basierend auf einem Ergebnis des Vergleichs, Bestimmen von Kontextinformation für die Schätzung der Patienten-Registrierung (85), und
- basierend auf der Kontextinformation, Erzeugen eines Steuersignals für ein oder mehrere mit dem chirurgischen Assistenzsystem assoziierte Komponenten.

Beispiel 25. Elektronische Datenverarbeitungsvorrichtung für ein chirurgisches Navigationssystem (899), umfassend mindestens einen Prozessor (21) und mindestens einen Speicher (22), wobei der mindestens eine Prozessor (21) eingerichtet ist, um Programmcode aus dem mindestens einen Speicher (22) zu laden und auszuführen, wobei der mindestens eine Prozessor (21) basierend auf dem Ausführen des Programmcodes die folgenden Schritte ausführt:
- basierend auf Referenzmarkern (892), die lagefest an einer an einem Skelett eines Patienten angebrachten Halterung (891) befestigt sind: Nachverfolgen (3105) einer Lage der Halterung (891) während eines Beobachtungszeitraums (69) in einem Referenz-Koordinatensystem (91),
   - basierend auf der Nachverfolgung der Lage der Halterung (891), Bestimmen (3110) einer Schätzung einer Patienten-Registrierung (85) im Referenz-Koordinatensystem (91) während des Beobachtungszeitraums (69),
   - Empfangen eines Steuersignals, welches Kontextinformation für die Schätzung der Patienten-Registrierung indiziert,
   - basierend auf der Kontextinformation, Anpassen der Schätzung der Patienten-Registrierung.

Beispiel 26. System, umfassend die elektronische Datenverarbeitungsvorrichtung nach Beispiel 24 sowie die elektronische Datenverarbeitungsvorrichtung nach Beispiel 25.

Beispiel 27. Computer-implementiertes Verfahren, das umfasst:
- Erhalten von intraoperativen Bilddaten, welche während eines Beobachtungszeitraums (69) mittels eines chirurgischen Visualisierungssystems (802, 809) erfasst sind, wobei das chirurgischen Visualisierungssystem in Bezug auf einen Patienten beweglich ist,
- basierend auf den intraoperativen Bilddaten, Bestimmen einer Position und/oder Orientierung einer in den intraoperativen Bilddaten dargestellten anatomischen Landmarke (75), wobei die anatomische Landmarke (75) in Bezug auf das Skelett lagefest ist,
- Durchführen eines Vergleichs, welcher eine Änderung einer Schätzung der Patienten-Registrierung (85) während des Beobachtungszeitraums (69) in Bezug setzt zu einer Änderung der Position und/oder Orientierung der anatomischen Landmarke (75) während des Beobachtungszeitraums (69),
- basierend auf einem Ergebnis des Vergleichs, Bestimmen von Kontextinformation für die Schätzung der Patienten-Registrierung (85), und
- basierend auf der Kontextinformation, Erzeugen eines Steuersignals für ein oder mehrere mit dem chirurgischen Assistenzsystem assoziierte Komponenten.

Selbstverständlich können die Merkmale der vorab beschriebenen Ausführungsformen und Aspekte der Erfindung miteinander kombiniert werden. Insbesondere können die Merkmale nicht nur in den beschriebenen Kombinationen, sondern auch in anderen Kombinationen oder für sich genommen verwendet werden, ohne das Gebiet der Erfindung zu verlassen.

Beispielsweise wurden voranstehend Techniken beschrieben, bei denen ein vom Operationsmikroskop getrenntes Navigationssystem verwendet wird, welches die Lage des Operationsmikroskops sowie eines Marker-Koordinatensystems in einem globalen Referenz-Koordinatensystem bestimmt. Allerdings sind verschiedene Variationen denkbar. Beispielsweise wäre es auch denkbar, dass eine entsprechende Sensorik für Navigationssystem im Operationsmikroskop positioniert ist, das heißt das Navigationssystem in das Operationsmikroskop integriert ist.

Ferner wurden voranstehend verschiedene Techniken beschrieben, bei denen eine Überlagerung von intraoperativen Bilddaten mit präoperativen Volumenbilddaten erfolgt. Es könnte auch eine Überlagerung von intraoperativen Bilddaten, die mittels unterschiedlicher Bildgebungsmodalitäten gewonnen werden, erfolgen. Beispielsweise könnten intraoperative Fluoreszenz-Bilddaten mit intraoperativen Bilddaten eines stereoskopischen Operationsmikroskops überlagert werden.

Ferner wurden voranstehend Techniken beschrieben, bei denen mehrere Komponenten zusammenwirken, um eine Schätzung für eine Patienten-Registrierung zu ermitteln und assoziierte Kontextinformation bzw. ein Steuersignal oder mehrere Steuersignale zu bestimmen. Die verschiedenen hierin beschriebenen Techniken gelten auch für die individuellen Komponenten. Das entsprechende Verhalten der individuellen Komponenten - beispielsweise ein Navigationssystem oder eine Datenverarbeitungsvorrichtung, die intraoperativen Bilddaten verarbeitet - ist entsprechend offenbart. Beispielsweise könnte eine Datenverarbeitungsvorrichtung eines Navigationssystem ein Steuersignal von einer anderen Datenverarbeitungsvorrichtung empfangen, wobei dieses Steuersignal indikativ für Kontextinformation für eine von der Datenverarbeitungsvorrichtung des Navigationssystems bestimmte Schätzung der Patienten-Registrierung ist. Die Datenverarbeitungsvorrichtung des Navigationssystems kann also basierend auf der Kontextinformation die Schätzung der Patienten-Registrierung anpassen. Entsprechende Techniken im Zusammenhang mit der Schätzung der Patienten-Registrierung und dem Empfangen eines entsprechenden Steuersignals wurden voranstehend im Zusammenhang mit Prozess 3001 in FIG. 4 diskutiert. Entsprechend ist auch die damit korrespondierende Datenverarbeitungsvorrichtung offenbart, welche das Steuersignal bzw. die Kontextinformation bestimmt. Entsprechende Techniken im Zusammenhang mit der Bestimmung der Kontextinformation wurden voranstehend beispielsweise im Zusammenhang mit FIG. 4: Prozess 3002 diskutiert.

## Patentansprüche

1. Chirurgisches Assistenzsystem (20, 802, 899) umfassend mindestens einen Prozessor (21) und mindestens einen Speicher (22), wobei der mindestens eine Prozessor (21) eingerichtet ist, um Programmcode aus dem mindestens einen Speicher (22) zu laden und auszuführen, wobei der mindestens eine Prozessor (21) basierend auf dem Ausführen des Programmcodes die folgenden Schritte ausführt:
- basierend auf Referenzmarkern (892), die lagefest an einer an einem Skelett eines Patienten angebrachten Halterung (891) befestigt sind: Nachverfolgen (3105) einer Lage der Halterung (891) während eines Beobachtungszeitraums (69) in einem Referenz-Koordinatensystem (91),
- basierend auf der Nachverfolgung der Lage der Halterung (891), Bestimmen (3110) einer Schätzung einer Patienten-Registrierung (85) im Referenz-Koordinatensystem (91) während des Beobachtungszeitraums (69),
- Erhalten von intraoperativen Bilddaten, welche während des Beobachtungszeitraums (69) mittels eines chirurgischen Visualisierungssystems (802, 809) erfasst sind, wobei das chirurgische Visualisierungssystem in Bezug auf den Patienten beweglich ist,
- basierend auf den intraoperativen Bilddaten, Bestimmen einer Schätzung einer Position und/oder Orientierung einer in den intraoperativen Bilddaten dargestellten anatomischen Landmarke (75) in dem Referenz-Koordinatensystem (91), wobei die anatomische Landmarke (75) in Bezug auf das Skelett lagefest ist,
- Durchführen eines Vergleichs der Schätzung der Position und/oder Orientierung der anatomischen Landmarke (75) mit einer vorgegebenen Referenz-Position und/oder Referenz-Orientierung, die aus Referenz-Bilddaten (60) des Patienten und anhand der Schätzung der Patienten-Registrierung (85) bestimmt ist,
- basierend auf einem Ergebnis des Vergleichs, Bestimmen von Kontextinformation für die Schätzung der Patienten-Registrierung (85), und
- basierend auf der Kontextinformation, Erzeugen eines Steuersignals für ein oder mehrere mit dem chirurgischen Assistenzsystem assoziierte Komponenten.

2. Chirurgisches Assistenzsystem nach Anspruch 1,
wobei die anatomische Landmarke (75) basierend auf einer vordefinierten Liste mit erlaubten und/oder verbotenen Kandidaten-Landmarken aufgefunden wird.

3. Chirurgisches Assistenzsystem nach Anspruch 2,
wobei die vordefinierte Liste basierend auf einem Typ des operativen Eingriffs bestimmt ist.

4. Chirurgisches Assistenzsystem nach Anspruch 3,
wobei zum Bestimmen der vordefinierten Liste verwendete Typinformation, welche indikativ für den Typ des operativen Eingriffs ist, mehrere Hierarchieebenen umfasst.

5. Chirurgisches Assistenzsystem nach Anspruch 4,
wobei eine erste Hierarchieebene der mehreren Hierarchieebenen eine Eingriffsregion des jeweiligen Typs des operativen Eingriffs spezifiziert,
wobei eine zweite Hierarchieebene der mehreren Hierarchieebenen eine Orientierung eines Zugangskanals innerhalb der Eingriffsregion für den jeweiligen Typ des operativen Eingriffs spezifiziert.

6. Chirurgisches Assistenzsystem nach einem der Ansprüche 2 bis 5,
wobei die vordefinierte Liste eine Priorisierung für die erlaubten und/oder verbotenen Kandidaten-Landmarken für einen jeweiligen Typ des operativen Eingriffs beinhaltet.

7. Chirurgisches Assistenzsystem nach einem der Ansprüche 2 bis 6,
wobei der mindestens eine Prozessor (21) basierend auf dem Ausführen des Programmcodes weiterhin den folgenden Schritt ausführt:
- Überprüfen (3211), ob die Anzahl der in der vordefinierten Liste vorhandenen erlaubten Kandidaten-Landmarken einen vorgegebenen Schwellenwert überschreitet, und
- wenn die Anzahl der erlaubten Kandidaten-Landmarken den vorgegebenen Schwellenwert nicht überschreitet: Ansteuern (3212) einer Benutzerschnittstelle mittels des Steuersignals zum Ausgeben einer Warnung.

8. Chirurgisches Assistenzsystem nach eine der voranstehenden Ansprüche,
wobei der mindestens eine Prozessor (21) basierend auf dem Ausführen des Programmcodes die folgenden Schritte ausführt: eine Detektion (3225) der anatomischen Landmarke in den intraoperativen Bilddaten, eine Lokalisierung (3235) in den intraoperativen Bilddaten basierend auf Vorwissen über ein Erscheinungsbild der anatomischen Landmarke, ein Tracking ein oder mehrerer anatomischer Landmarken über der Zeit, einer topographischen Rekonstruktion (3240) einer Oberfläche der anatomischen Landmarke, ein Posen-Schätzungsmodell zum Bestimmen einer Form und Lage der anatomischen Landmarke, und eine Umrechnung (3245) der Position und/oder Orientierung der anatomischen Landmarke in das Referenz-Koordinatensystem oder ein weiteres Referenz-Koordinatensystem.

9. Chirurgisches Assistenzsystem nach einem der voranstehenden Ansprüche,
wobei der mindestens eine Prozessor (21) weiterhin eingerichtet ist, um basierend auf dem Ausführen des Programmcodes den folgenden Schritt auszuführen:
- Einstellen eines Schwellenwerts (219) für den Vergleich in Abhängigkeit von ein oder mehreren Kriterien, wobei die ein oder mehreren Kriterien einen Typ des operativen Eingriffs und/oder eine Vorhersage für einen gegenwärtigen oder nachfolgenden Operationsschritt des operativen Eingriffs umfassen.

10. Chirurgisches Assistenzsystem nach einem der voranstehenden Ansprüche,
wobei die Kontextinformation ortsaufgelöst für einer Umgebung der anatomischen Landmarke (75) und/oder anhand von Szeneninformation bestimmt wird.

11. Chirurgisches Assistenzsystem nach einem der voranstehenden Ansprüche,
wobei der mindestens eine Prozessor (21) weiterhin eingerichtet ist, um basierend auf dem Ausführen des Programmcodes den folgenden Schritt auszuführen:
- basierend auf der Kontextinformation: Ansteuern einer Benutzerschnittstelle zum Indizieren von lokalen Bereichen mit besonders hoher oder niedriger Konfidenz für die Schätzung der Patienten-Registrierung (85).

12. Chirurgisches Assistenzsystem nach einem der voranstehenden Ansprüche,
wobei die Kontextinformation eine alternative Schätzung der Patienten-Registrierung (85) und/oder eine Unsicherheit für die Schätzung der Patienten-Registrierung (85) umfasst.

13. Chirurgisches Assistenzsystem nach eine der voranstehenden Ansprüche,
wobei der mindestens eine Prozessor (21) basierend auf dem Ausführen des Programmcodes weiterhin den folgenden Schritt ausführt:
- Ansteuern eines Navigationssystem basierend auf dem Steuersignal.

14. Chirurgisches Assistenzsystem nach eine der voranstehenden Ansprüche, wobei der mindestens eine Prozessor (21) basierend auf dem Ausführen des Programmcodes weiterhin den folgenden Schritt ausführt:
- Suchen ein oder mehrerer vorgegebener Kandidaten-Landmarken in den intraoperativen Bilddaten, und
- wenn das Suchen der ein oder mehreren vorgegebenen Kandidaten-Landmarken für eine vorgegebenen Zeitdauer erfolglos verbleibt, Anfordern (3231) einer Erweiterung eines Gesichtsfelds des chirurgischen Visualisierungssystems.

15. Computer-implementiertes Verfahren, das umfasst:
- basierend auf Referenzmarkern (892), die lagefest an einer an einem Skelett eines Patienten angebrachten Halterung (891) befestigt sind: Nachverfolgen (3105) einer Lage der Halterung (891) während eines Beobachtungszeitraums (69) in einem Referenz-Koordinatensystem (91),
- basierend auf der Nachverfolgung der Lage der Halterung (891), Bestimmen (3110) einer Schätzung einer Patienten-Registrierung (85) im Referenz-Koordinatensystem (91) während des Beobachtungszeitraums (69),
- Erhalten von intraoperativen Bilddaten, welche während des Beobachtungszeitraums (69) mittels eines chirurgischen Visualisierungssystems (802, 809) erfasst sind, wobei das chirurgischen Visualisierungssystem in Bezug auf den Patienten beweglich ist,
- basierend auf den intraoperativen Bilddaten, Bestimmen einer Schätzung einer Position und/oder Orientierung einer in den intraoperativen Bilddaten dargestellten anatomischen Landmarke (75) in dem Referenz-Koordinatensystem (91), wobei die anatomische Landmarke (75) in Bezug auf das Skelett lagefest ist,
- Durchführen eines Vergleichs der Schätzung der Position und/oder Orientierung der anatomischen Landmarke (75) mit einer vorgegebenen Referenz-Position und/oder Referenz-Orientierung, die aus Referenz-Bilddaten (60) des Patienten und anhand der Schätzung der Patienten-Registrierung (85) bestimmt ist,
- basierend auf einem Ergebnis des Vergleichs, Bestimmen von Kontextinformation für die Schätzung der Patienten-Registrierung (85), und
- basierend auf der Kontextinformation, Erzeugen eines Steuersignals für ein oder mehrere mit einem chirurgischen Assistenzsystem assoziierte Komponenten.
